# EUROPÄISCHE PATENTSCHRIFT

(11) **EP 1 590 317 B1**
(45) Veröffentlichungstag und Bekanntmachung des Hinweises auf die Patenterteilung: **24.01.2007**
(21) Anmeldenummer: 03815696.4
(22) Anmeldetag: 22.12.2003
(51) Int. Cl.: C07C 227/18, C07C 229/40, C07C 229/76, C07B 53/00, A61K 51/04

(54) **ENANTIOMERENREINES (4S,8S)- UND (4R, 8R)-4-P-NITROBENZYL-8-METHYL-3, 6, 9-TRIAZA- SP 3 /SP N, SP 6 /SP N, SP 9 /SP N-TRICARBOXYMETHYL-1, 11-UNDECANDISÄURE UND DEREN ABKÖMMLINGE, VERFAHREN ZU DEREN HERSTELLUNG UND VERWENDUNG ZUR HERSTELLUNG PHARMAZEUTISCHER MITTEL**
ENANTIOMER-PURE (4S,8S)- AND (4R, 8R)-4-P-NITROBENZYL-8-METHYL-3, 6, 9-TRIAZA- SP 3 /sp N, SP 6 /SP N, SP 9 /SP N-TRICARBOXYMETHYL-1, 11-UNDECANOIC ACID AND DERIVATIVES THEREOF, METHOD FOR PRODUCING THEM, AND THEIR USE FOR PRODUCING PHARMACEUTICAL AGENTS
ACIDES (4S,8S)-4-P-NITROBENZYL-8-METHYL-3,6,9-TRIAZA- SP 3 /SP N, SP 6 /SP N, SP 9 /SP N-TRICARBOXYMETHYL-1,11-UNDECANOIQUE ET (4R,8R)-4-P-NITROBENZYL-8-METHYL-3,6,9-TRIAZA- SP 3 /SP N, SP 6 /SP N, SP 9 /SP N-TRICARBOXYMETHYL-1,11-UNDECANOIQUE ENANTIOMERIQUEMENT PURS ET DERIVES DE CEUX-CI, PROCEDES

(30) Priorität: 04.02.2003 DE 10305463
(43) Veröffentlichungstag der Anmeldung: 02.11.2005
(73) Patentinhaber: Schering Aktiengesellschaft, 13353 Berlin (DE)
(72) Erfinder: LEHMANN, Lutz, 13357 Berlin (DE); FRIEBE, Matthias, 13509 Berlin (DE); BRUMBY, Thomas, 10589 Berlin (DE); SÜLZLE, Detlev, 10589 Berlin (DE); PLATZEK, Johannes, 12621 Berlin (DE)
(74) Vertreter: Teipel, Stephan
(86) Internationale Anmeldenummer: PCT/EP2003/014921
(87) Internationale Veröffentlichungsnummer: WO 2004/069787

(56) Entgegenhaltungen:
- WO-A-02/067859
- CLARK H. CUMMINS ET.AL.: "A CONVENIENT SYNTHESIS OF BIFUNKTIONAL CHELATING AGENTS BASED ON DIETHYLENETRIAMINEPENTAACTETIC ACID AND THEIR COORDINATION CHEMISTRY WITH YTTRIUM (III)" BIOCONJUGATE CHEM., Bd. 2, 1991, Seiten 180-186, XP001181242
- HISATAKA KOBAYASHI ET.AL.: "AVIDIN-DENDRIMER-(1B4M-Gd)254: A TUMOR TARGETING THERAPEUTIC AGENT FOR GADOLINIUM NEUTRON CAPTURE THERAPY OF INTRAPERITONEAL DISSEMINATED TUMOR WHICH CAN BE MONITORED BY MRI" BIOCONJUGATE CHEM., Bd. 12, 2001, Seiten 587-593, XP002279279
- HISATAKA KOBAYASHI ET.AL.: "MONOCLONAL ANTIBODY-DENDRIMER CONJUGATES ENABLE REDIOLABELING OF ANTIBODY WITH MARKELY HIGH SPECIFIC ACTIVITY WITH MINIMAL LOSS OF IMMUNOREACTIVITY" EUROPEAN JOURNAL OF NUCLEAR MEDICINE, Bd. 27, Nr. 9, 2000, Seiten 1334-1339, XP002279280

## Beschreibung

Die Erfindung betrifft die in den Patentansprüchen gekennzeichneten Gegenstände, das heißt, (4S,8S)- und (4R,8R)-4-p-Nitrobenzyl-8-methyl-3,6,9-triaza-³N,⁶N,⁹N-tricarboxymethyl-1,11-undecandisäure und deren Abkömmlinge, Verfahren zu deren Herstellung und deren Verwendung zur Herstellung pharmazeutischer Mittel für die Radiodiagnostik, Radiotherapie oder NMR-Diagnostik.

Die Anwendung von Radiopharmaka für diagnostische und therapeutische Zwecke ist seit langem im Bereich der biologischen und medizinischen Forschung bekannt. Insbesondere werden Radiopharmaka dazu benutzt, um bestimmte Strukturen wie beispielsweise das Skelett, Organe oder Gewebe darzustellen. Die diagnostische Anwendung setzt den Gebrauch solcher radioaktiver Mittel voraus, die sich nach Applikation spezifisch in den Strukturen im Patienten anreichern, die untersucht werden sollen. Diese sich lokal anreichernden radioaktiven Mittel können dann mittels geeigneter Detektoren, wie beispielsweise Szintillationskameras oder anderer geeigneter Aufnahmeverfahren, aufgespürt, aufgenommen oder szintigraphiert werden. Die Verteilung und relative Intensität des detektierten radioaktiven Mittels kennzeichnet den Ort einer Struktur, in dem sich das radioaktive Mittel befindet, und kann die Anwesenheit von Anomalien in Strukturen und Funktionen, pathologische Veränderungen etc. darstellen.

In ähnlicher Weise können Radiopharmaka als therapeutische Mittel dem Patienten verabreicht werden, um bestimmte krankhafte Gewebe oder Bereiche zu bestrahlen. Solche Behandlung erfordert die Herstellung radioaktiver therapeutischer Mittel, die sich in bestimmten Strukturen, Organen oder Geweben anreichern.
Ein von der Firma IDEC Pharmaceuticals Corp. entwickeltes Radiopharmakon zur Therapie des non-Hodgkin-Lymphoms stellt Zevalin^{®} dar (s. z.B. *Cancer* **(2002)** Feb 15; 94, (4 Suppl):1349-57). Strahlendes Ion ist hierbei β-emittierendes ⁹⁰Y, welches durch einen Chelator (Methylsubstituiertes-Diethylen-Triamin-Penta-Essigsäure-Derivat (MX-DTPA)) an einen Tumorspezifischen Antikörper gebunden sind.

Die kernmagnetische Resonanz (NMR) ist heute eine breit angewandte, für die in vivo-Bildgebung ausgenutzte Methode der medizinischen Diagnostik, mit der über die Messung der magnetischen Eigenschaften der Protonen im Körperwasser Körpergefäße und Körpergewebe (einschließlich Tumore) dargestellt werden können. Es werden hierzu z.B. Kontrastmittel eingesetzt, die durch Beeinflussung bestimmter NMR-Parameter der Körperprotonen (z.B. der Relaxationszeiten T¹ und T²) eine Kontrastverstärkung in den resultierenden Bildern bewirken bzw. diese Bilder erst lesbar machen. Vor allem kommen Komplexe paramagnetischer Ionen, wie z.B. Gadolinium-enthaltende Komplexe (z.B. Magnevist^{®}) aufgrund des Effekts der paramagnetischen Ionen auf die Verkürzung der Relaxationszeiten zur Anwendung.

Sowohl paramagnetische Ionen, wie z.B.: Gd³⁺, Mn²⁺, Cr³⁺, Fe³⁺ und Cu²⁺ als auch viele metallische Radionuklide können nicht in freier Form als Lösungen verabreicht werden, da sie hoch toxisch sind. Um diese Ionen für eine *in vivo-*Anwendung geeignet zu machen, werden sie in der Regel komplexiert. Beispielsweise wird in der EP-A-0 071 564 u.a. das Megluminsalz des Gadolinium(III)-Komplexes der Diethylentriaminpentaessigsäure (DTPA) als Kontrastmittel für die NMR-Tomographie beschrieben. Ein Präparat, das diesen Komplex enthält, wurde unter dem Namen Magnevist^{®} weltweit als erstes NMR-Kontrastmittel zugelassen. Dieses Kontrastmittel verteilt sich nach intravenöser Applikation extrazellulär und wird durch glomeruläre Sekretion renal ausgeschieden. Eine Passage intakter Zellmembranen wird praktisch nicht beobachtet. Magnevist^{®} ist besonders gut für die Darstellung pathologischer Bereiche (z.B. Entzündungen, Tumore) geeignet.

Die bekannten Radiotherapeutika und Kontrastmittel sind jedoch nicht für alle Anwendungsfälle befriedigend einzusetzen. So verteilen sich viele dieser Mittel im gesamten extrazellulären Raum des Körpers. Um die Effizienz dieser Mittel in der in vivo-Diagnostik und der Therapie zu steigern, wird versucht, deren Spezifität und Selektivität beispielsweise zu Targetzellen oder gewünschten Bereichen und Strukturen des Körpers zu erhöhen. Eine Verbesserung dieser Eigenschaften ist beispielsweise durch Kopplung der Metallkomplexe an Biomoleküle nach dem "Drug-Targeting"-Prinzip zu erreichen. Als Biomoleküle bieten sich Plasmaproteine, Antikörper, deren Fragmente, Hormone, Wachstumsfaktoren und Substrate von Rezeptoren und Enzymen (z.B. WO 97/12850, Institut für Diagnostikforschung an der FU Berlin). Doch bislang ist z.B. die Tumorspezifität (Tumoranreicherung) in vielen Fällen noch nicht ausreichend hoch, was besonders bei der Radioimmuntherapie ein wichtiges Ziel ist.

Es ist weiterhin wünschenswert, Mittel für die Diagnostik und Therapie zur Verfügung zu stellen, die neben einer möglichst hohen Zielspezifität eine große *in vivo*-Stabilität für die zumeist toxischen komplexierten Metallionen besitzen.

Eine Aufgabe der Erfindung war es daher, neue Mittel für die Radio- und NMR-Diagnostik sowie die Radiotherapie zur Verfügung zu stellen, die die genannten Nachteile nicht aufweisen und insbesondere eine hohe in vivo-Stabilität, gute Verträglichkeit und vor allem organspezifische Eigenschaften aufweisen. Einerseits soll die Retention in den zu untersuchenden Tumorgeweben oder Organen ausreichend sein, um bei geringer Dosierung die für eine effiziente Diagnose und Therapie notwendige Qualität von Bildern bzw. ausreichende Bestrahlung zu erzielen. Andererseits aber soll anschließend eine möglichst schnelle und weitestgehend vollständige Ausscheidung der Metalle aus dem Körper gewährleistet sein. Ferner sollen die NMR-Kontrastmittel eine hohe Protonen-Relaxivität zeigen und damit bei einer Steigerung der Signalintensität eine Reduzierung der Dosis erlauben.
Es wurden verschiedene Versuche unternommen, die Eigenschaften von biokoppelbaren DTPA -Derivaten durch die Einführung von Substituenten zu verbessern.

Brechbiel et al. beschreiben z.B. eine detaillierte Synthese methylsubstituierter DTPA-Derivate, die beispielsweise an Antikörper gekoppelt werden können ("A Convenient Synthesis of Bifunctional Chelating Agents Based on Diethylenetriaminepentaacetic Acid and Their Coordination Chemistry with Yttrium", Bionconjugate Chemistry, (1991), 180-186. "Synthesis of (1-(p-isothiocyanatobenzyl) derivatives of DTPA and EDTA. Antibody Labeling and Tumor-Imaging Studies.", Inorg. Chem. (1986), 25, 2772-2781.). In der Patentanmeldung WO 88/01618 von Gansow et al. wird DTPA, welches mit einem Methylsubstituenten in Position 8 und einem *para*-funktionalisierten Benzylsubstituenten in Position 4 versehen ist, offenbart.

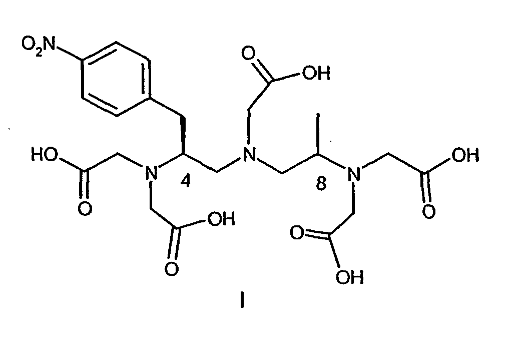

Die Verbindung I wurde MX-DTPA, mx-DTPA oder auch 1B4M-H₅DTPA genannt.
Die Patentanmeldung WO01/41743 der Firma IDEC Pharmaceuticals Corporation beschreibt eine *regio*selektive Synthese der Verbindung l, die von Boc-geschütztem (S)-p-Nitrophenylalanin und einem monogeschützten Diamin ausgeht. Das stereogene Zentrum in Position 4 dieser Verbindung wird als (S)-konfiguriert beschrieben; das stereogene Zentrum in Position 8 wird nicht definiert.

Wie oben schon erwähnt, ist MX-DTPA Bestandteil des Präparates ZEVALIN^{®} für die Behandlung des non-Hodgkin-Lymphoms. Auch hier wird als chelatisierender Ligand das Gemisch aus (4S,8R)- und (4S,8S)-MX-DTPA eingesetzt.
McMurry et al. (J. Med. Chem., (**1998**), 41, 3546-3549.) konnten zeigen, dass Cyclohexyl-substituierte DTPA, die mit Nitrobenzyl substituiert sind, auf Grund der rigiden und sperrigen Struktur des Cyclohexanrings eine bevorzugte Konfiguration besitzen. So weist Verbindung II eine höhere *in-vivo* Stabilität als 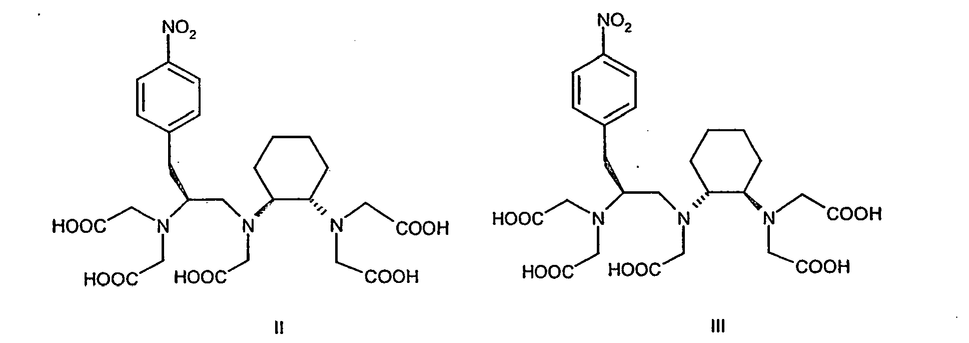 Verbindung III auf.

Obwohl MX-DTPA (I) gut untersuchte und akzeptable Eigenschaften - z.B. erhöhte Komplexstabilität verglichen mit der unsubstituierten DTPA - zeigte, bleibt es dennoch wünschenswert, die Metallkomplexstabilität *in vivo* weiter zu erhöhen und Mittel für die Diagnose und die Therapie zur Verfügung zu stellen, die eine möglichst hohe Sicherheit aufweisen.

Es wurde nun gefunden, dass MX-DTPA-Derivate, bei denen der verhältnismäßig kleine Methylsubstituent (Position 8) in enantioselektiver Weise eingeführt worden ist, bei geeigneter Konfiguration überraschenderweise eine deutlich höhere thermodynamische Stabilität *in vitro* aufweist als das Diastereomerengemisch (IV) (s.u.).

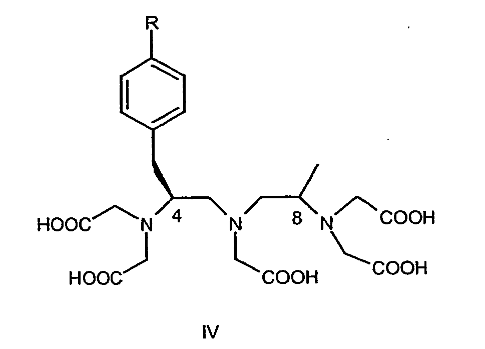

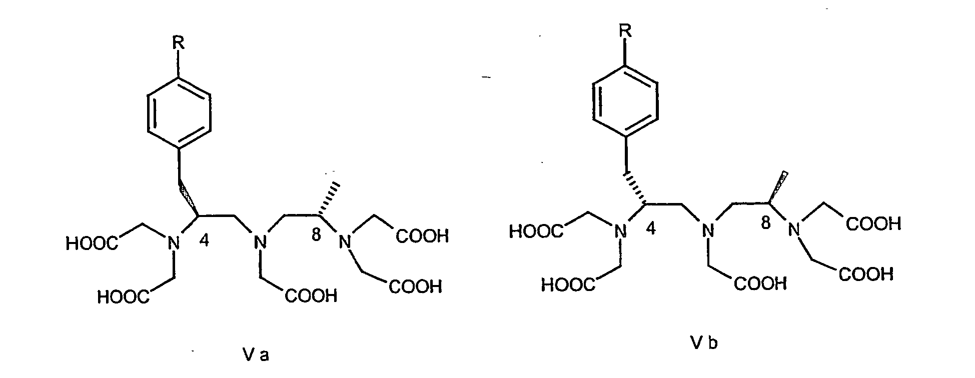

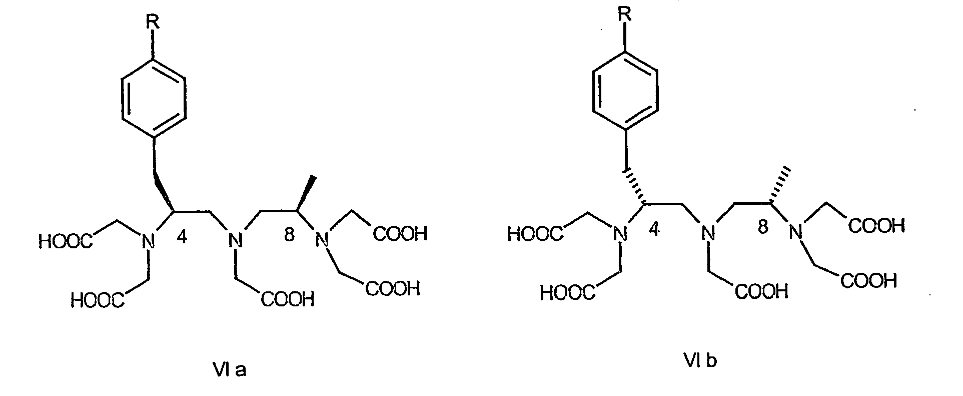

Dabei muss, wie herausgefunden wurde, die Konfiguration von Methyl- und Benzylsubstituent als (4S,8S) (s. Verbindung Va) oder (4R,8R) (s. Verbindung Vb) vorliegen, um den beschriebenen positiven Effekt zu erreichen. Die Konfigurationen (4S,8R) oder (4R,8S) (s. Verbindung VI a bzw. b) führen dagegen zu verminderter Komplexstabilität. Ein beeindruckendes beispielhaftes Experiment konnte zeigen, dass sich in einer Mischung von je einem Äquivalent Va (R = -NO2), VIa (R = -NO2) und Gd(III)-Salz fast ausschließlich der Gd-Komplex der Verbindung Va bildet. Außerdem konnte die thermodynamische Stabilitätskonstante für Verbindung Va mit IogK_{Y} = 24,7 ± 0,7 bestimmt werden, die somit deutlich erhöht ist gegenüber der Stabilitätskonstante des Diastereomerengemischs IV (IogK_{Y} = 22,5 (J. Med. Chem. (1998), 41, 3546-3549)). Es wurde zudem gefunden, dass die Radiotoxiziät des Metallkomplexes der Verbindung Va in vivo gegenüber der Verbindung VIa erniedrigt ist.

Dieses Ergebnis ist unerwartet und überraschend, da es sich beim Methylsubstituenten in Position 8 um keine rigide, "raumorganisierende" oder sterisch anspruchsvolle Struktur handelt, so wie es beim Cyclohexylsubstituent der Verbindungen II und III der Fall ist. So war zu erwarten gewesen, dass alle vier Stereoisomere nahezu gleiche Komplexierungseigenschaften aufweisen würden. Wie bereits beschrieben zeigte sich aber, dass im Vergleich die erfindungsgemäßen Verbindungen Va und Vb die deutlich besseren Komplexierungseigenschaften als die Verbindungen VIa und VIb besitzen.

Darüber hinaus zeigen die erfindungsgemäßen Verbindungen eine gute Relaxivität und gute Wasserlöslichkeit, so dass sie sich als pharmazeutische Mittel insbesondere für die Radio- und NMR-Diagnostik sowie Radiotherapie eignen.

Die Erfindung betrifft somit Verbindungen der allgemeinen Formel VIIa und VIIb 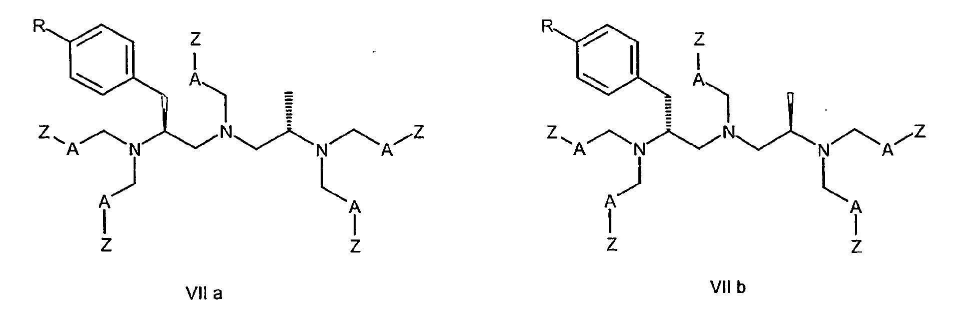 worin
- Z: für ein Wasserstoffatom oder ein Metallionenäquivalent eines Elements der Ordnungszahl 21-29, 31, 32, 37-39, 42-44, 46, 47, 49, 58-71, 75, 77, 82 oder 83 steht,
- A: für eine Gruppe -COO- steht,
- R: für eine Nitrogruppe, eine Aminogruppe, oder eine andere funktionelle Gruppe, die mit einem Biomolekül verknüpft werden kann, oder für einen geradkettigen oder verzweigten, gesättigten oder ungesättigten und gegebenenfalls durch ein bis sechs O-Atome, bzw. Phenylen,
-NHCO-, -CONH-, 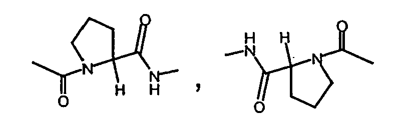
und/oder -NH-(C=S)-NH-Gruppen unterbrochenen C₁ - C₂₅ -Alkylrest, der gegebenenfalls an einer beliebigen Stelle mit ein bis sechs Carboxylgruppen, Hydroxylgruppen, Aminogruppen oder anderen funktionellen Gruppen substituiert ist, sowie deren Salze mit organischen oder anorganischen Basen mit den Maßgaben, dass der Alkylrest mindestens eine funktionelle Gruppe, die mit einem Biomolekül verknüpft werden kann, enthält und dass mindestens zwei Z für ein Metallionenäquivalent stehen.

Der Rest R kann für einen Alkylrest mit 1 - 25 Kohlenstoffatomen stehen (wobei er mindestens eine funktionelle Gruppe enthält). Dieser Alkylrest kann geradkettig oder verzweigt, gesättigt oder ungesättigt sein (z.B.: 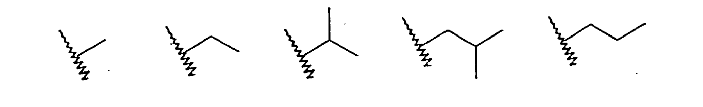 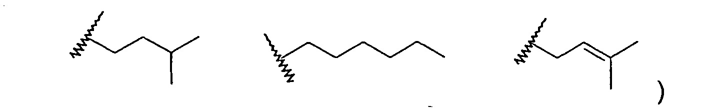 und ist an einer beliebigen Stelle mit ein bis sechs Carboxyl-, Hydroxyl-, und/oder Aminogruppen (z.B.: 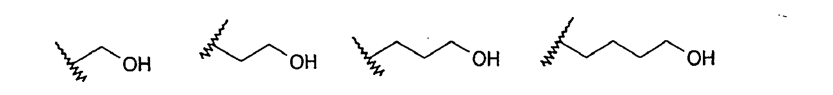 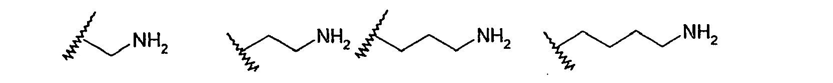 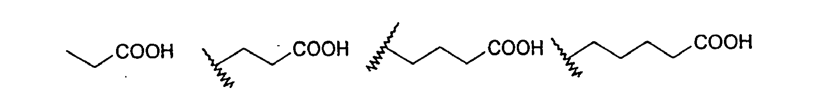 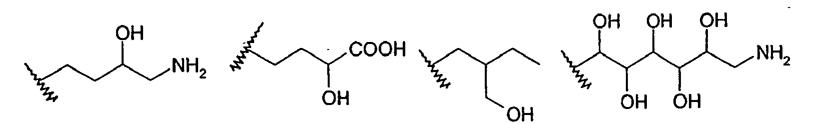 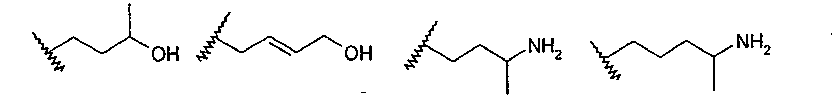 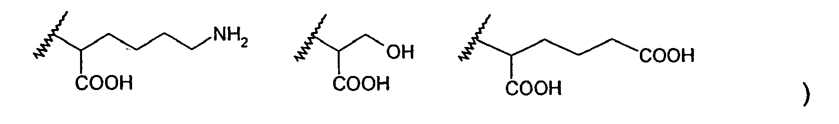 oder mindestens einer anderen funktionellen bindenden Gruppe versehen, die mit einem Biomolekül verknüpft werden kann - wie z.B. Garboxyl, aktiviertem Carboxyl, Amino, Nitro, Isocyanat, Isothiocyanat, Hydrazin, Semicarbazid, Thiosemibarbazid, Chloracetamid, Bromacetamid, Iodacetamid, Acryl, Acylamino, gemischten Anhydriden, Azid, Säurechlorid, Hydroxid, Sulfonylchlorid, Vinylsulphon, Carbodiimid, Maleimid, Dioxo oder einer anderen funktionellen bindenden Gruppe (z.B. 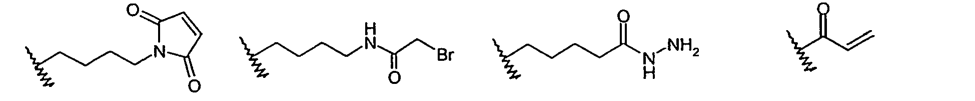 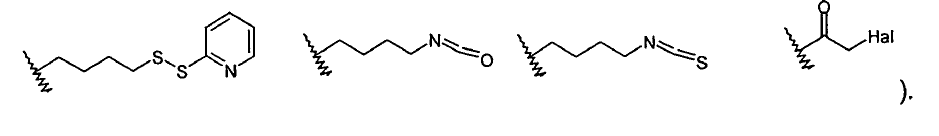 Der C₁ - C₂₅ Alkylrest kann gegebenenfalls durch ein bis sechs O-Atome, Phenylen-, -NHCO-, -CONH-, -O-(CO)-NH- 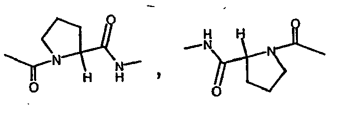 und/oder -NH-(C=S)-NH-Gruppen unterbrochen sein (z.B.: 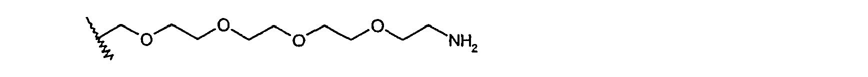 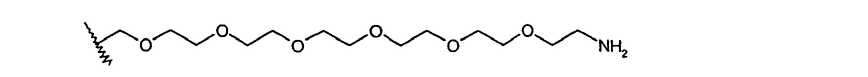 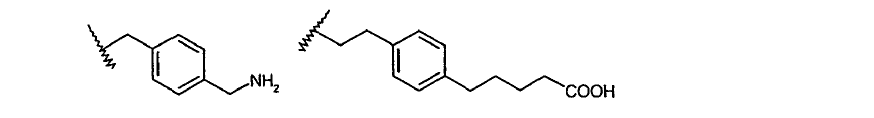 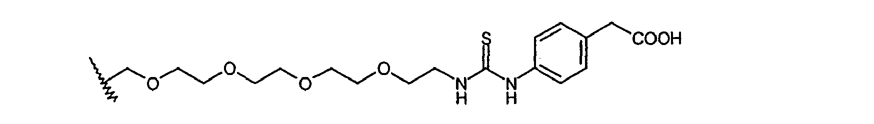 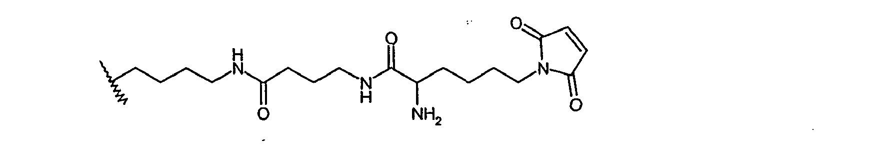 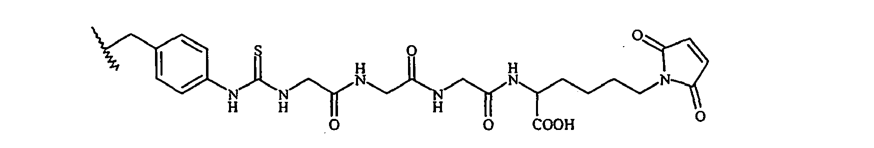 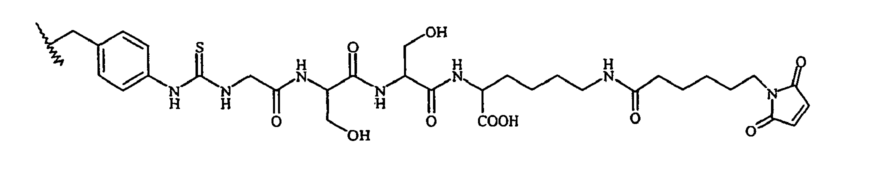 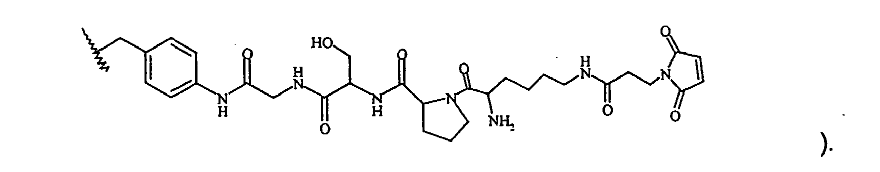 R kann auch für eine funktionelle Gruppe selbst stehen, wie z.B. Carboxyl, aktiviertes Carboxyl, Amino, Nitro, Isocyanat, Isothiocyanat, Hydrazin, Semicarbazid, Thiosemibarbazid, Chloracetamid, Bromacetamid, Iodacetamid, Acryl, Acylamino, gemischten Anhydriden, Azid, Säurechlorid, Säurebromid, Hydroxid, Sulfonylchlorid, Vinylsulphon, Carbodiimid, Maleimid oder Diazo (z.B.: 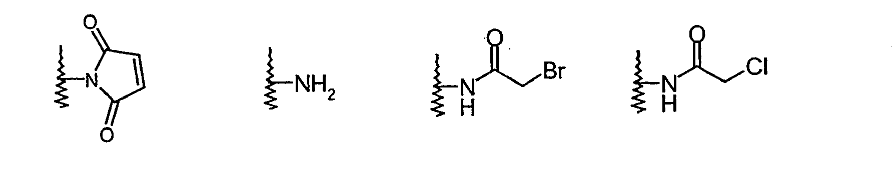 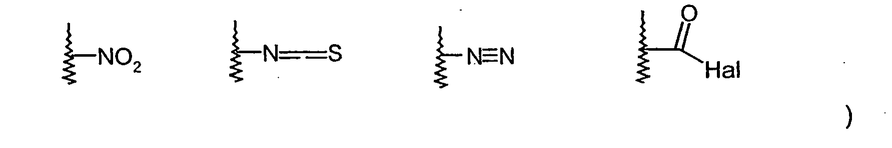 oder einer anderen funktionellen bindenden Gruppe.

Eine Vielzahl der oben genannten möglichen R-Substituenten gestattet eine selektive Umsetzung mit funktionellen Gruppen der Biomoleküle im optimalen pH-Bereich, beispielsweise die Addition an -SH-Gruppen (Cystein im Biomolekül), und zwar ausschließlich von -SH-Gruppen, z.B. an Maleimide ((2,5-Dioxo-2,5-dihydro-pyrrol-1-yl)-Verbindungen, s.o.) oder Bromacetamide, wenn die Kopplung im schwach sauren pH-Bereich stattfindet.

Unter aktivierter Carboxylgruppe werden vorstehend solche Carboxylgruppen verstanden, die so derivatisiert sind, daß sie die Reaktion mit einem Biomolekül erleichtern. Welche Gruppen zur Aktivierung verwendet werden können, ist bekannt und es kann beispielsweise auf M. und A. Bodanszky, "The Practice of Peptide Synthesis", Springerverlag 1984 verwiesen werden. Beispiele sind Adukte der Carbonsäure mit Carbodiimiden oder aktivierter Ester wie z.B. Hydroxybenzotriazolester. Besonders bevorzugt wird die aktivierte Carboxylgruppe für X ausgewählt aus 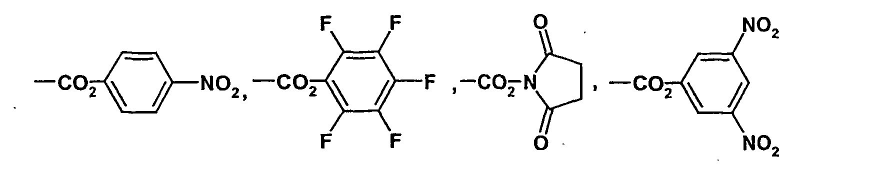 und

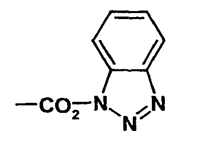

Die aktivierten Ester der vorstehend beschriebenen Verbindungen werden wie dem Fachmann bekannt hergestellt. Für den Fall von Isothiocyanaten oder α-Halogenacetaten werden die entsprechenden terminalen Aminvorstufen nach literaturbekannten Methoden mit Thiophosgen oder 2-Halo-Essigsäure-Halogeniden umgesetzt. Auch die Umsetzung mit entsprechend derivatisierten Estern von N-Hydroxysuccinimid wie beispielsweise: 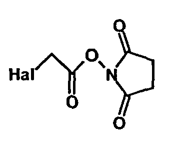 ist möglich (Hal = Halogen).

Allgemein können für diesen Zweck alle üblichen Aktivierungsmethoden für Carbonsäuren verwendet werden, die im Stand der Technik bekannt sind. Enthält die R-Substituent eine Amidgruppe, so wird diese beispielsweise hergestellt, indem eine aktivierte Carbonsäure mit einem Amin umgesetzt wird. Die Aktivierung der Carbonsäure erfolgt nach den üblichen Methoden. Beispiele für geeignete Aktivierungsreagentien sind Dicyclohexylcarbodiimid (DCC), 1-Ethyl-3-(3-dimethylaminopropyl)-carbodiimid-hydrochlorid (EDC), Benzotriazol-1-yloxytris(dimethylamino)-phosphoniumhexafluorophosphat (BOP) und O-(Benzotriazol-1-yl)-1,1,3,3-tetramethyluroniumhexafluorophosphat (HBTU), vorzugsweise DCC. Auch der Zusatz von O-nukleophilen Katalysatoren, wie z.B. N-Hydroxysuccinimid (NHS) oder N-Hydroxybenzotriazol ist möglich.

Handelt es sich beim Substituenten um eine Carbonsäurefunktion, so kann diese in geschützter Form (z.B. in Form des Benzylesters) eingesetzt werden, die Abspaltung der Schutzgruppe kann dann hydrogenolytisch erfolgen.

Um diese Carbonsäurefunktion an eine geeignete funktionelle Gruppe eines geeigneten Biomoleküls (zur Beschreibung von Biomolelülen: s.u.) zu knüpfen, sollte diese im Regelfall zunächst aktiviert werden. Bevorzugt werden dazu aktivierte Ester intermediär erzeugt, welche dann von einer nukleophilen Gruppe des Biomoleküls angegriffen werden. Auf diese Weise entsteht eine kovalente Verknüpfung zwischen dem Biomolekül und der Verbindung der Formel I. Bevorzugte aktivierte Ester sind die Ester des N-Hydroxysuccinimids, die Ester des Paranitrophenols oder die Ester des Pentafluorphenols. Soll die funktionelle Gruppe in Form eines Isothiocyanats an das Biomolekül geknüpft werden, so wird bevorzugt zunächst ein terminales Amin verwendet, welches, wenn notwendig, mit einer geeigneten Schutzgruppe versehen sein kann. Geeignete Schutzgruppen sind aus der Peptidchemie bekannt. Nach Abspaltung der Schutzgruppe kann durch Umsetzung des primären terminalen Amins mit Thiophosgen das Isothiocyanat erzeugt werden. An dieses können nukleophile Gruppen des Biomoleküls addiert werden.

Die Synthese der Konjugate erfolgt in der Regel derart, daß zunächst ein derivatisierter und funktionalisierter Ligand oder Chelatkomplex erzeugt wird, welcher dann an das Biomolekül geknüpft wird. Es ist aber auch möglich, daß im Falle der Verwendung von synthetisch hergestellten Biomolekülen der erfindungsgemäße Ligand oder Chelatkomplex während der Synthese des Biomoleküls in dieses eingebaut wird. Dies kann beispielsweise während der sequentiellen Synthese von Oligopeptiden am Syntheseroboter erfolgen. Falls erforderlich, können dazu die in der Synthese des entsprechenden Biomoleküls üblichen Schutzgruppen in die erfindungsgemäße Verbindung eingeführt werden. Diese werden dann im Zuge der üblichen Synthesealgorithmen am Synthesizer wieder abgespalten.

Die erfindungsgemäßen Verbindungen enthalten mindestens zwei Chiralitätszentren (Position 4 und 8). Auch R kann ein oder mehrere zusätzliche Chiralitätszentren enthalten, wobei in den Beschreibungen und den Ansprüchen nicht zwischen den verschiedenen Enantiomeren unterschieden wird, doch umfassen die genannten Verbindungen immer beide Enantiomeren und bei Vorliegen mehrerer Stereozentren auch alle möglichen Diastereomeren sowie deren Mischungen.

Unter "Biomolekül" wird vorliegend jedes Molekül verstanden, das entweder natürlich beispielsweise im Körper auftritt oder mit analoger Struktur synthetisch hergestellt wurde. Darüber hinaus werden hierunter solche Moleküle verstanden, die mit einem biologisch, beispielsweise im Körper auftretenden Molekül oder einer dort auftretenden Struktur in Wechselwirkung treten können, so daß sich beispielsweise die Konjugate an bestimmten, gewünschten Stellen des Körpers anreichern. Unter "Körper" wird vorliegend jeder pflanzliche oder tierische Körper verstanden, wobei tierische und insbesondere menschliche Körper bevorzugt sind.

Biomoleküle sind insbesondere die in Lebewesen auftretenden Moleküle, die als Produkte einer evolutionären Selektion durch geordnetes und komplexes Zusammenwirken spezifische Aufgaben für den Organismus erfüllen und die Grundlage seiner Lebensfunktionen (Stoff- und Formwechsel, Fortpflanzung, Energiehaushalt) ausmachen. In Biomolekülen sind zumeist aus einfachen Bausteinen (Aminosäuren, Nucleobasen, Monosacchariden, Fettsäuren, etc.) größere Moleküle (Proteine, Nucleinsäuren, Polysaccharide, Lipide, etc.) aufgebaut. Entsprechende Makromoleküle werden auch als Biopolymere bezeichnet.

Vorteilhaft kann das Biomolekül beispielsweise ein Polypeptidgerüst aus Aminosäuren mit Seitenketten aufweisen, die mit der reaktiven Gruppe der erfindungsgemäßen Verbindungen eine Reaktion eingehen können. Solche Seitenketten schließen beispielsweise die Carboxylgruppen von Asparaginsäure- und Glutaminsäureresten, die Aminogruppen von Lysinresten, die aromatischen Gruppen von Tyrosin- und Histidinresten und die Sulphhydrylgruppen von Cysteinresten ein.

Eine Übersicht über Biomoleküle mit zahlreichen Beispielen findet sich in dem Skriptum "Chemie der Biomoleküle" der TU-Graz (H. Berthold et al., Institut für Organische Chemie, TU-Graz, 2001), das auch über das Internet unter www.orgc.tu-graz.ac.at eingesehen werden kann. Der Inhalt dieses Dokuments wird durch Bezugnahme in die vorliegende Beschreibung aufgenommen.

Zur Bildung der erfindungsgemäßen Konjugate sind folgende Biomoleküle besonders geeignet:
Biopolymere, Proteine, wie Proteine, die eine biologische Funktion haben, HSA, BSA, etc., Proteine und Peptide, die sich an bestimmten Stellen im Organismus anreichern (z.B. an Rezeptoren, Zellmembranen, Kanälen etc.), durch Proteasen spaltbare Peptide, Peptide mit synthetischen Sollbruchstellen (z.B. labile Ester, Amide etc.), Peptide, die durch Metalloprotheasen gespalten werden, Peptide mit photospaltbaren Linkern, Peptide mit oxydativen Mitteln (Oxydasen) spaltbaren Gruppen, Peptide mit natürlichen und unnatürlichen Aminosäuren, Glycoproteine (Glycopeptide), Signal-Proteine, antivirale Proteine und Apoktosis, synthetisch modifizierte Biopolymere, wie mit Linkern derivatisierte Biopolymere, modifizierte Metalloproteasen und derivatisierte Oxydase etc., Kohlenhydrate (Mono- bis Polysaccharide), wie derivatisierte Zucker, im Organismus spaltbare Zucker, Cyclodextrine und dessen Derivate, Aminozucker, Chitosan, Polysulfate und Acetylneuraminsäure-Derivate, Antikörper, wie monoklonale Antikörper, Antikörperfragmente, polyklonale Antikörper, Minibodies, Single Chains (auch solche, die mit Linkern zu mehrfachen Fragmenten verknüpft sind), rote Blutkörperchen und andere Blutbestandteile, Cancermarker (z.B. CAA) und Zell-Adhäsions-Stoffe (z.B. Lewis X und Anti-Lewis X-Derivate), DNA und RNA Fragmente, wie derivatisierte DNAs und RNAs (z.B. solche, die durch das SELEX-Verfahren gefunden wurden), synthetische RNA und DNA (auch mit unnatürlichen Basen), PNAs (Hoechst) und Antisense, β-Aminosäuren (Seebach), Vektoramine zur Einschleusung in die Zelle, biogene Amine, Pharmazeutika, onkologische Präparate, synthetische Polymere, die auf ein biologisches Target (z.B. Rezeptor) gerichtet sind, Steroide (natürliche und modifizierte), Prostaglandine, Taxol und dessen Derivate, Endotheline, Alkaloide, Folsäure und deren Derivate, bioaktive Lipide, Fette, Fettsäureester, synthetisch modifizierte Mono-, Di- und Triglyceride, Liposome, die an der Oberfläche derivatisiert sind, Micellen aus natürlichen Fettsäuren oder aus Perfluoralkyl-Verbindungen, Porphyrine, Texaphrine, erweitere Porphyrine, Cytochrome, Inhibitoren, Neuramidasen, Neuropeptide, Immunomodulatoren, wie FK 506, CAPE und Gliotoxin, Endoglycosidasen, Substrate, die durch Enzyme aktiviert werden wie Calmodolin Kinase, Casein-Kinase II, Gluthathion-S-Transferase, Heparinase, Matrix-Metalloprotheasen, β-Insulin-Rezeptor-Kinase, UDP-Galactose 4-Epimerase, Fucosidasen, G-Proteine, Galactosidasen, Glycosidasen, Glycosyltransferasen und Xylosidase, Antibiotika, Vitamin und Vitamin-Analoga, Hormone, DNA-Interkalatoren, Nucleoside, Nucleotide, Lektine, Vitamin B12, Lewis-X und Verwandte, Psoralene, Dientrienantibiotika, Carbacycline, VEGF (vascular endothelial growth factor), Somatostatin und dessen Derivate, Biotin-Derivate, Antihormone, tumorspezifische Proteine und Synthetika, Polymere, die sich in sauren oder basischen Bereichen des Körpers anreichern (pHgesteuerte Verteilung), Myoglobine, Apomyoglobine etc., Neurotransmitter-Peptide, Tumornecrosefaktoren, Peptide, die sich in entzündetem Gewebe anreichern, Bloodpool-Reagenzien, Anionen und Kationen-Transporterproteine, Polyester (z.B. der Milchsäure), Polyamide und Polyphosphate.

Die meisten der vorgenannten Biomoleküle sind kommerziell beispielsweise bei Merck, Alderich, Sigma, Calibochem oder Bachem erhältlich.

Außerdem können als Biomoleküle alle in der WO 96/23526 und der WO 01/08712 offenbarten "Plasmaproteinbindungsgruppen" bzw. "Zielbindungsgruppen" eingesetzt werden. Der Inhalt dieser beiden Offenlegungsschriften wird daher durch Bezugnahme in die vorliegende Beschreibung aufgenommen.

Die Anzahl der erfindungsgemäßen Verbindungen der Formel VII a oder VII b pro Biomolekül ist prinzipiell beliebig, bevorzugt ist jedoch ein molekulares Verhältnis 0,3:1 bis 11:1, insbesondere von 0,5:1 bis 7:1.

Ferner eignen sich die Verbindungen der Formel VIIa und b zur Konjugation an all diejenigen Moleküle, welche im Stand der Technik mit Fluoreszenzfarbstoffen umgesetzt werden, um beispielsweise ihre Lokalisation durch Epifluoreszenzmikroskopie innerhalb der Zelle zu bestimmen. Auch können die Verbindungen mit prinzipiell beliebigen Medikamenten konjugiert werden, um dann nach Verabreichung des Medikaments den Transport innerhalb des Organismus durch die NMR- oder Szintigraphie-Technik zu verfolgen. Ferner ist es möglich, daß die erfindungsgemäßen Konjugate aus den Verbindungen der Formel VII a und b und den Biomolekülen weitere zusätzliche Moleküle enthalten, die an die Biomoleküle konjugiert worden sind. Mit dem Begriff "Biomolekül" im Sinne der Erfindung sind also alle Moleküle umfasst, die in biologischen Systemen vorkommen und alle Moleküle, die biokompatibel sind (Definition Biomoleküle s.a. oben).

Die Relaxivität der erfindungsgemäßen paramagnetischen Komplexe ist so hoch, dass sie sich besonders gut für die NMR-Diagnostik eignen.
Die erfindungsgemäßen Verbindungen binden an Proteine. Diese Eigenschaft ermöglicht es ihnen, gebunden an Plasmaproteine, länger im Blutstrom zu verweilen und so eine Darstellung des Vasalraumes zu ermöglichen. Darüber hinaus wird auch eine Darstellung von Stellen erhöhter Permeabilität, wie sie beispielsweise in Tumoren zu finden ist, möglich. Diese erhöhte Gefäßpermeabilität ist weiterhin Grundlage der Tumortherapie mit radioaktiven Metallkomplexen. Das Pharmakon verlässt innerhalb des Tumors das Gefäß, bleibt im Gewebe und setzt dieses seiner therapeutisch wirksamen Strahlung aus.
Die Plasmaproteinbindung ermöglicht auch eine bildgebende Diagnostik zur Lokalisation von Infarkten oder Nekrosen infolge der Anreicherung der erfindungsgemäßen Substanzen im Infarkt oder der Nekrose.

Detektion, Lokalisierung und Überwachung von Nekrosen oder Infarkten ist ein wichtiger Bereich in der Medizin. So resultiert der Myokardinfarkt nicht sofort in einem unwiederbringlich funktionsuntüchtigen Gewebe, sondern leitet einen dynamischen Prozeß ein, der sich über einen längeren Zeitraum (Wochen bis Monate) erstreckt. Die Erkrankung verläuft in etwa drei Phasen, die nicht scharf voneinander getrennt, sondern überlappend sind. Die erste Phase, die Entwicklung des Myokardinfarktes, umfaßt die 24 Stunden nach dem Infarkt, in denen die Zerstörung wie eine Stoßwelle (Wellenfrontphänomen) vom Subendokard zum Myokard fortschreitet. Die zweite Phase, der bereits bestehende Infarkt, umfasst die Stabilisierung des Bereiches, in dem Faserbildung (Fibrose) als Heilprozeß erfolgt. Die dritte Phase, der ausgeheilte Infarkt, beginnt, nachdem alles zerstörte Gewebe durch fibröses Narbengewebe ersetzt ist. Während dieser Periode findet eine umfangreiche Restrukturierung statt.

Für die Beurteilung eines Myokardinfarktes ist es von entscheidender Bedeutung, zu wissen, wie groß der Anteil des bei dem Infarkt definitiv verlorenen Gewebes ist und an welcher Stelle der Verlust erfolgte, denn von dieser Kenntnis hängt die Art der Therapie ab.
Infarkte erfolgen nicht nur im Myokard, sondern auch in anderen Geweben wie im Hirn oder in der Niere.
Während der Infarkt in gewissem Umfang heilbar ist, können bei einer Nekrose, dem lokal begrenzten Gewebetod, nur die schädlichen Folgen für den Restorganismus verhindert oder wenigstens gemildert werden. Nekrosen können auf vielfache Weise entstehen: durch Verletzungen, Chemikalien, Sauerstoffdefizit oder durch Strahlung. Wie beim Infarkt ist die Kenntnis von Umfang und Art einer Nekrose wichtig für das weitere ärztliche Vorgehen.

Die Herstellung der erfindungsgemäßen Verbindungen der allgemeinen Formeln VIIa und b erfolgt dadurch, dass man in an sich bekannter Weise bei den Verbindungen der allgemeinen Formel VII'a und VII'b 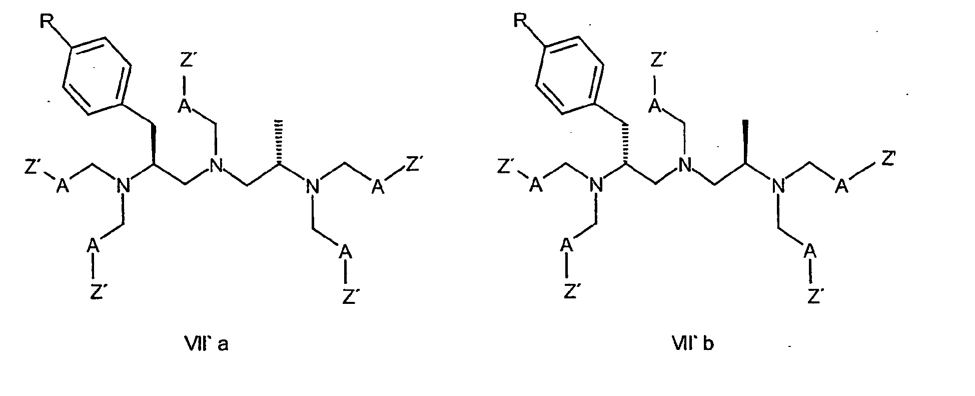 worin Z' eine Carboxylschutzgruppe bedeutet, die Schutzgruppen Z' abspaltet und die so erhaltenen Säuren in an sich bekannter Weise mit mindestens einem Metalloxid oder Metallsalz eines Elements der Ordnungszahlen 21-29, 31, 32, 37-39, 42-44, 46, 47, 49, 58-71, 75, 77, 82 oder 83 umsetzt und anschließend, falls gewünscht, vorhandene acide Wasserstoffatome mit anorganischen und/oder organischen oder Aminosäuren in physiologisch verträgliche Salze überführt.

Als Carboxylschutzgruppen Z' kommen niedere Alkyl-, Aryl- und Aralkylgruppen infrage, beispielsweise die Methyl-, Ethyl-, Propyl-, Butyl-, Phenyl-, Benzyl-, Diphenylmethyl-, Triphenylmethyl-, Bis(4-nitrophenyl)-methylgruppe sowie Trialkylsilylgruppen.

Die Abspaltung der Schutzgruppen Z' erfolgt in an sich bekannter Weise, beispielsweise durch Hydrolyse, alkalische Verseifung der Ester, vorzugsweise mit Alkali in wässrig -alkoholischer Lösung bei Temperaturen von 0° C bis 50° C oder im Falle von Benzylestern durch katalytische Hydrierung und im Falle von t-Butylestern durch saure Hydrolyse, beispielsweise mit Salz- oder Trifluoressigsäure (Protective Groups in Organic Synthesis, 2^{nd} Edition, T.W. Greene and P.G. M. Wutz, John Wiley & Sons, Inc., New York, 1991).

Die Herstellung der erfindungsgemäßen Verbindungen der Formel VII a und VII b wird im folgenden am Beispiel der ausgewählten Verbindung 1, in der Z' t- 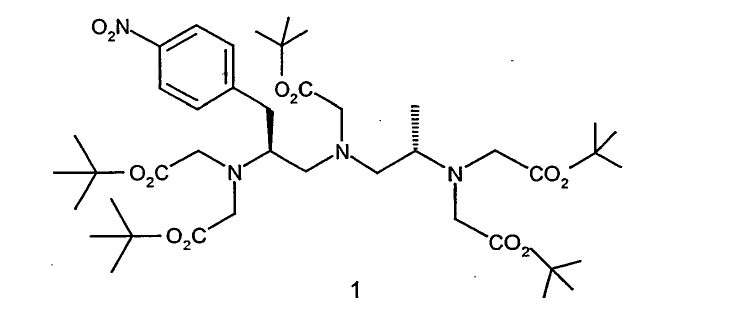 Butyl ist, erläutert.

Durch alkalische Verseifung und anschließende Ionenaustauscherbehandlung kann die Verbindung 1 in die Verbindung VII' a mit freien Carbonsäuren überführt werden.

Verbindung 1 entsteht aus Triamin 2 durch eine Alkylierungsreaktion mit Bromessigsäure-tert-butylester in Acetonitril/Wasser-Gemisch mit Kaliumcarbonat als Base.

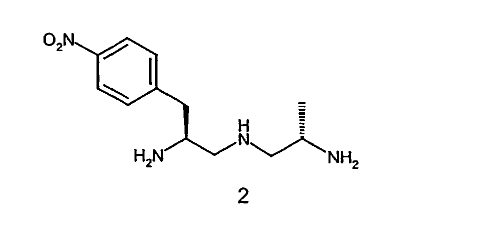

Die Verbindung 2 ist durch Reduktion des Amides 3 mit Boran-Tetrahydrofurankomplex zugänglich. Man folgt dabei den Bedingungen, wie sie 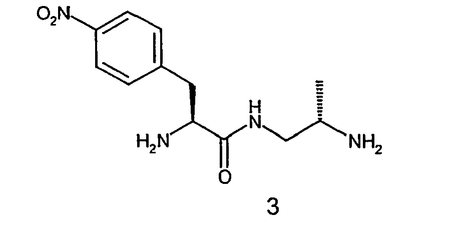 beispielsweise in *J*. *Amer. Chem. Soc*., (**1990**), 9608 beschrieben werden.

Das Amid 3 wird aus dem zweifach Boc-geschützten Diamin 4 durch Umsetzen mit Trifluoressigsäure und Dichlormethan hergestellt.

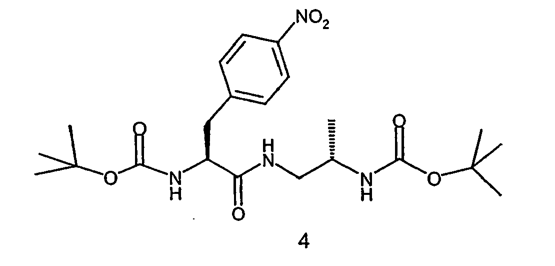

Die Bildung des Amides 4 erfolgt dabei nach den dem Fachmann wohlbekannten Methoden, beispielsweise der Säure-aktivierung durch
- Oxalylchlorid: J. Org. Chem., 29: 843 (1964)
- Thionylchlorid: Helv., 42: 1653 (1959)
- Carbodiimide. Helv. 46: 1550 (1963)
- Carbodiimide/Hydroxysuccinimid:J. Am. Chem. Soc. 86: 1839 (1964) sowie J. Org. Chem. 53: 3583 (1988); Synthesis 453 (1972)
- Anhydridmethode:, 2-Ethoxy-1-ethoxycarbonyl-1,2-dihydrochinolin:J. Am. Chem. Soc. 90:1651 (1986); Int. J. Pept. Prot. Res., 26:493 (1985); Am. Soc. 73:3547(1951)
- Imidazolid-Methode: Am. Soc.91:2691 (1969)
J. Med. Chem.1996, 392596; Tetrahedron Letters 1994, 35, 5981; Bioorg. Med. Letters 1996, 6, 55; J. Chem. Soc. Commun. 1994, 201,
aus der kommerziell erhältlichen Säure 5 und dem monogeschützten Amin 6.

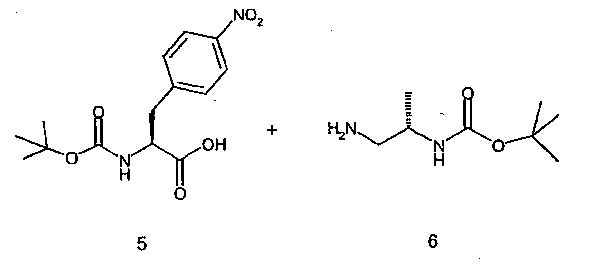

Die Verbindung 6 ergibt sich durch Reduktion des Azids 7 mit Wasserstoff und Pd/C in Essigester.

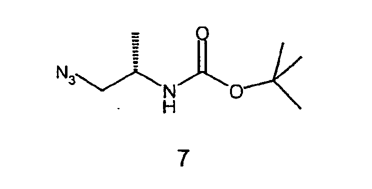

Verbindung 7 geht aus der Substitutionsreaktion von Mesylat 8 mit Natriumazid hervor.

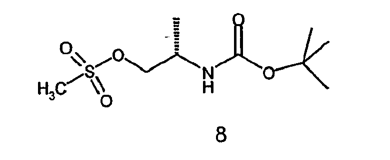

Das Mesylat 8 kann durch Umsetzung mit Alkohol 9 und Methansulfonsäurechlorid erhalten werden.

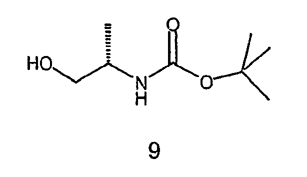

Alkohol 9 ist das Produkt aus einer Umsetzung von komerziell erhältlichem (S)-2-Amino-1-propanol (10) und Di-*tert*-butyldicarbonat [(Boc)₂O] in Tetrahydrofuran.

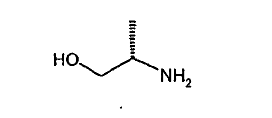

Die in Verbindung 1 enthaltene Nitrogruppe dient nach ihrer Umwandlung in die Amino-gruppe unmittelbar als Bindungsstelle für Biomoleküle, beispielsweise über eine Amidbildung mit Hilfe aktivierter Ester oder reduktive Aminierung mit Carbonylgruppen oder nach Umwandlung in selektiv reagierende Gruppen. Diese Umwandlungsreaktionen sind dem Fachmann wohlbekannt.
So beschreiben Gansow (EP 484984) und Meares (US 4622420) die Darstellung von Halogenacetamiden von acyclischen Komplexbildnern, die zur Kopplung mit -SH-Gruppen oder -NH₂₋-Gruppen Verwendung finden.

Eine selektive Kopplung mit Aminogruppen ermöglicht die Isothiocyanatogruppe. Ihre Darstellung und die Umsetzung mit Aminen zu den entsprechenden Thioharnstoffen ist beispielsweise in dem Patent US 4680338 von Immunomedics beschrieben worden. Die Umsetzung mit Hydraziden zu den den Thio-semicarbaziden wird in der Anmeldung WO 95/15335 der Neorx Corp. geschildert..
Eine selektive Umsetzung mit -SH-Gruppen ermöglichen Maleimide. Ihre Darstellung und Umsetzung werden beispielsweise in den Patenten US 5273743 und EP 446071 von Hybritech oder in EP 345723 von Nihon-Medi Physics beschrieben.

Die Einführung der gewünschten Metallionen von Komplexen für die Herstellung von NMR-Diagnostika kann in der Weise erfolgen, wie sie in den Patentschriften EP 71564, EP 130934 und DE-OS 34 01 052 offenbart worden ist. Dazu wird das Metalloxid oder ein Metallsalz (beispielsweise ein Chlorid, Nitrat, Acetat, Carbonat oder Sulfat) des gewünschten Elements in Wasser und/oder einem niederen Alkohol (wie Methanol, Ethanol oder Isopropanol) gelöst oder suspendiert und mit der Lösung oder Suspension der äquivalenten Menge des erfindungsgemäßen Komplexbildners umgesetzt.
Die Neutralisation eventuell noch vorhandener freier Carboxygruppen erfolgt mit Hilfe anorganischer Basen (z.B. Hydroxyden, Carbonaten oder Bicarbonaten) von z.B. Natrium, Kalium, Lithium, Magnesium oder Calcium und/oder organischer Basen wie unter anderem primärer, sekundärer und tertiärer Amine, wie z.B. Ethanolamin, Morpholin, Glucamin, N-Methyl- und N,N-Dimethylglucamin, sowie basischer Aminosäuren, wie z.B. Lysin, Arginin und Ornithin oder von Amiden ursprüngliche neutraler oder saurer Aminosäuren.

Zur Herstellung der neutralen Komplexverbindungen kann man beispielsweise in sauren Komplexsalzen in wässriger Lösung oder Suspension soviel der gewünschten Base zusetzen, dass der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z.B. niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen,
Sollen die Komplexbildner zur Herstellung von Radiodiagnostika oder - therapeutika Verwendung finden, kann die Herstellung der Komplexe aus den Komplexbildnern nach den in "Radiotracers for Medical Applications", Vol I, CRC Press, Boca Raton, Florida (1983) beschriebenen Methoden erfolgen.

Es kann wünschenswert sein, den Komplex erst kurz vor seiner Verwendung herzustellen, insbesondere, wenn er als Radiopharmakon eingesetzt werden soll. Daher umfasst die Erfindung auch einen Kit zur Herstellung von Radiopharmaka, umfassend eine Verbindung der Formel VIIa oder VIIb, worin Z für ein Radioisotop steht.
Gegenstand der Erfindung sind ferner pharmazeutische Mittel, die mindestens eine physiologisch verträgliche Verbindung der allgemeinen Formel VIIa oder VIIb enthalten, gegebenenfalls mit den in der Galenik üblichen Zusätzen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wässrigem Medium suspendiert oder löst, und anschließend die Suspension oder Lösung gegebenenfalls sterilisiert. Geeignete Zusätze sind beispielsweise physiologisch unbedenkliche Puffer (wie z.B. Tromethamin), Zusätze von Komplexbildnern oder schwachen Komplexen (wie z.B. Diethylentriaminpentaessigsäure oder die zu den erfindungsgemäßen Metallkomplexen korrespondierenden Ca-Komplexe) oder - falls erforderlich - Elektrolyte wie z.B. Natriumchlorid oder - falls erforderlich - Antioxidantien wie z.B. Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [z.B. Methylcellulose, Lactose, Mannit] und/oder Tensid(en) [z.B. Lecithine, Tween^{®}, Myrj^{®}] und/oder Aromastoff(en) zur Geschmackskorrektur [z.B. ätherischen Ölen] gemischt.

Prinzipiell ist es auch möglich, die erfindungsgemäßen pharmazeutischen Mittel auch ohne Isolierung der Komplexsalze herzustellen. In jedem Fall muss besondere Sorgfalt darauf verwendet werden, die Chelatbildung so vorzunehmen, dass die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1 fMol-1,3 Mol/l des Komplexsalzes und werden in der Regel in Mengen von 0,5 pMol/kg-5 mMol/kg dosiert. Sie sind zur enteralen und parenteralen Applikation bestimmt. Die erfindungsgemäßen Komplexverbindungen kommen zur Anwendung
1. für die NMR-Diagnostik in Form ihrer Komplexe mit den paramagnetischen Ionen der Elemente mit den Ordnungszahlen 21-29, 42, 44 und 58-70. Geeignete Ionen sind beispielsweise das Chrom(III)-, Eisen(II)-, Cobalt(II), Nickel(II)-, Kupfer(II)-, Praseodym(III)-, Neodym(III)-, Samarium(III)- und Ytterbium(III)-ion. Wegen ihres starken magnetischen Moments sind für die NMR-Diagnostik besonders bevorzugt das Gadolinium(III)-, Terbium(III), Dysprosium(III)-, Holmium(III)-, Erbium(III)-Mangan(II)- und Eisen(III)-ion.
2. für die Radiodiagnostik und Radiotherapie in Form ihrer Komplexe mit den Radioisotopen der Elemente mit den Ordnungszahlen 26, 27, 29, 31, 32, 37-39, 43, 46, 47, 49, 61, 62, 64, 67, 70, 71, 75, 77, 82 und 83.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Mittel nicht nur eine hohe Stabilität *in vitro* auf, sondern auch eine überraschend hohe Stabilität in vivo, so dass eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001-5 mMol/kg, vorzugsweise 0,005-0,5 mMol/kg, dosiert. Details der Anwendung werden z.B. in H.-J. Weinmann et al., *Am. J. of Roentgenology* 142, 619 (1984) diskutiert.

Niedrige Dosierungen (unter 1 mg/kg Körpergewicht) von organspezifischen NMR-Diagnostika sind zum Beispiel zum Nachweis von Tumoren und von Herzinfarkt einsetzbar. Besonders niedrige Dosierungen der erfindungsgemäßen Komplexe sind für die Anwendung in der Radiotherapie und Radiodiagnostik geeignet. So kommen sowohl für therapeutische wie auch diagnostische Zwecke Dosierungen von 0,5 pM/kg - 5 µM/kg, vorzugsweise 50 pM/kg - 500 nMol/kg zur Anwendung. Üblicherweise werden hinsichtlich des radioaktiven Metallions ca. 100 - 100.000-fach geringere molare Konzentrationen verwendet als dies für die Chelatoren bzw. Chelator-Biokonjugate der Fall ist, so dass also die Chelatoren bzw. Chelator-Biokonjugate im Überschuss vorliegt.

Ferner können die erfindungsgemäßen Komplexverbindungen vorteilhaft als Suszeptibilitäts-Reagenzien und als *shift*-Reagenzien für die *in vivo*-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika und Radiotherapeutika geeignet. Details ihrer Anwendung und Dosierung werden z.B. in "Radiotracers for Medical Applications", CRC-Press, Boca Raton, Florida, 1983, sowie in Eur. J. Nucl. Med. 17 (1990) 346-364 und Chem. Rev. 93 (1993) 1137-1156 beschrieben.

Für SPECT geeignet sind die Komplexe mit den Isotopen ¹¹¹In und ^{99m}Tc.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co, ⁶⁸Ga, ⁶⁴Cu, ⁸⁶Y und ^{94m}Tc verwendet (Heiss, W.D.; Phelps, M.E.; Positron Emission Tomography of Brain, Springer Verlag Berlin, Heidelberg, New York 1983).

Die erfindungsgemäßen Verbindungen sind überraschenderweise auch zur Differenzierung von malignen und benignen Tumoren in Bereichen ohne Blut-Hirn-Schranke geeignet.

Sie zeichnen sich auch dadurch aus, dass sie vollständig aus dem Körper eliminiert werden und somit gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei, die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei der Strahlentherapie von malignen Tumoren (z.B. Ausnutzen von Mössbauer-Effekten oder bei Neutroneneinfangtherapie).

Geeignete β-emittierende Ionen sind z.B. ⁴⁶Sc, ⁴⁷Sc, ⁴⁸Sc, ⁷²Ga, ⁷³Ga, ⁹⁰Y, ⁶⁷Cu, ¹⁰⁹Pd, ¹¹¹Ag, ¹⁴⁹Pm, ¹⁵³Sm, ¹⁶⁶Ho, ¹⁷⁷Lu, ¹⁸⁶Re und ¹⁸⁸Re, wobei ⁹⁰Y, ¹⁷⁷Lu, ⁷²Ga, ¹⁵³Sm und ⁶⁷Cu bevorzugt werden. Geeignete geringe Halbwertzeiten aufweisende □-emittierende Ionen sind z.B. ²¹¹At, ²¹¹Bi, ²¹²Bi, ²¹³, Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R. L. Mills et al. [Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in vivo-Applikation der erfindungsgemäßen therapeutischen Mittel können diese zusammen mit einem geeigneten Träger wie z.B. Serum oder physiologischer Kochsalzlösung und zusammen mit einem anderen Protein wie z.B. Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden vorzugsweise parenteral, bevorzugt i.v., appliziert.

Details der Anwendungen von Radiotherapeutika werden z.B. in R. W. Kozak et al. TIBTEC, Oktober 1986, 262, diskutiert (s. a. Bioconjugate Chem. 12 (2001) 7-34).

Insgesamt ist es gelungen, neue Komplexbildner, Metallkomplexe und Metallkomplexsalze zu synthetisieren, die verbesserte Möglichkeiten in der diagnostischen und therapeutischen Medizin erschließen.

Die nachfolgenden Beispiele dienen zur näheren Erläuterung des Erfindungsgegenstands:

Die Bespiele 10, 12 - 15, 18, 19, 20, 21, 28 - 31 beschreiben Konjugate mit Antikörpern. Konjugate mit anderen Biomolekülen können nach folgenden allgemeinen Arbeitsvorschriften hergestellt werden:
Hierin steht "AAV" für allgemeine Arbeitsvorschrift, "RP-18" bezeichnet eine "reversed phase" stationäre Chromatographiephase. Die Anzahl der Komplexe pro Biomolekül wurde mittels Szinitiegraphie oder ICP (inductively coupled plasma atomic emission spectroscopy) bestimmt.

### Allgemeine Arbeitsvorschrift (AAV) I: Albumin-Amid-Konjugate

3 mmol der Säure werden in 15 mL DMF gelöst, unter Eiskühlung mit 380 mg (3,3 mmol) N-Hydroxysuccinimd und 681 mg Dicyclohexylcarbodiimid versetzt und 1 Stunde im Eis voraktiviert. Die Aktivestermischung wird innerhalb von 30 Minuten in eine Lösung von 16,75 g (0,25 mmol) Rinderserumalbumin (BSA) in 150 mL Phosphatpuffer (pH 7,4) eingetropft und 2 Stunden bei Raumtemperatur gerührt. Die Ansatzlösung wird filtriert, das Filtrat über eine AMICON^{®} YM30 (cut off 30.000 Da) ultrafiltriert, das Retentat über eine Sephadex^{®} G50 -Säule chromatographiert und die Produktfraktionen gefriergetrocknet.

### Allgemeine Arbeitsvorschrift (AAV) II: Albumin-Maleimid-Konjugate

0,0438 mmol des Maleimids in 1 mL DMF werden zu 0,84 g (0,0125 mmol) Rinderserumalbumin (BSA), gelöst in 15 mL Phosphatpuffer (pH 7,4), gegeben und eine Stunde bei Raumtemperatur gerührt. Die Ansatzlösung wird filtriert, das Filtrat über eine AMICON^{®} YM30 (cut off 30.000 Da) ultrafiltriert, das Retentat über eine Sephadex^{®} G50 -Säule chromatographiert und die Produktfraktionen gefriergetrocknet.

### Allgemeine Arbeitsvorschrift (AAV) III: Herstellung von Amid-Konjugaten

3 mmol der Säure werden in 15 mL DMF gelöst, unter Eiskühlung mit 380 mg (3,3 mmol) N-Hydroxysuccinimd und 681 mg Dicyclohexylcarbodiimid versetzt und 1 Stunde im Eis voraktiviert. Die Aktivestermischung wird in eine Lösung von 2,5 mmol Aminkomponente in 15-150 mL DMF eingetropft und über Nacht bei Raumtemperatur gerührt. Die Ansatzlösung wird filtriert und an Kieselgel chromatographiert.

### Allgemeine Arbeitsvorschrift (AAV) IV: Herstellung von Maleimido-SH-Konjugaten

3 mmol des Maleimids in 15 mL DMF werden zu 2,5 mmol SH-Komponente in 15-150 mL DMF eingetropft und eine Stunde bei Raumtemperatur gerührt. Die Ansatzlösung wird filtriert, das Filtrat über eine AMICON^{®} YM30 (cut off 30.000 Da) ultrafiltriert, das Retentat über eine Sephadex^{®} G50 -Säule chromatographiert und die Produktfraktionen gefriergetrocknet.

### Allgemeine Arbeitsvorschrift (AAV) V: Herstellung von Halogenacetamido-SH-Konjugaten

3 mmol des Halogenacetamids in 15 mL DMF werden zu 2,5 mmol SH-Komponente in 15-150 mL DMF eingetropft und acht Stunden bei Raumtemperatur gerührt. Die Ansatzlösung wird filtriert, das Filtrat über eine AMICON^{®} YM30 (cut off 30.000 Da) ultrafiltriert, das Retentat über eine Sephadex^{®} G50 -Säule chromatographiert und die Produktfraktionen gefriergetrocknet.

### Beispiel 1

### a) (S)-(2-Hydroxy-1-methyl-ethyl)-carbaminsäure tert-butyl ester

10,50 g (140 mmol) (S)-(+)-2-Amino-1-propanol wurden in 110 ml THF gelöst und bei 0°C gekühlt. Zu dieser gerührten Lösung wurde eine Lösung von 30,2 (139 mmol) Di-tert.-butyldicarbonat in 45 ml THF hinzugetropft. Die Reaktionsmischung wurde für 90 Minuten bei 25°C gerührt und am Rotationsverdampfer eingeengt. Der Rückstand wurde in 300 ml Diethylether aufgenommen und anschließend mit 90 ml 0,01 M HCl-Lösung gewaschen. Die organische Phase wurde mittels Natriumsulfat getrocknet und am Rotationsverdampfer und an der Ölpumpe eingeengt. Das Rohprodukt (20,4 g) wurde ohne weitere Reinigung für die folgende Reaktion eingesetzt.

### b) (S)-Methansulfonsäure 2-tert-butoxycarbonylamino-propyl ester

20,3 g (116 mmol) 1a wurden in 125 ml Dichlormethan gelöst und mit 17,7 g (175 mmol) Triethylamin versetzt. Es wurde bei 0°C ein Lösung von 14,7 g (128 mmol) Methansulfonsäurechlorid in 30 ml Dichlormethan hinzugetropft. Die Reaktionslösung wurde bei 0°C für 2 h gerührt und anschließend mit 300 ml Wasser versetzt. Die organische Phase wurde abgetrennt. Die wässrige Phase wurde zweimal mit 150 ml Dichlormethan extrahiert. Die vereinigten organischen Phasen wurden einmalig mit 150 ml 0,1 M HCI-Lösung, zweimal mit je 150 ml 5% iger Natriumhydrogencarbonatlösung und abschließend mit 50 ml wässriger, gesättigter Natriumchloridlösung gewaschen. Die organische Phase wurde mit Natriumsulfat getrocknet. Die Lösung wurde eingeengt und im Kühlschrank auskristallisiert. Die Kristalle wurden mit kaltem Hexan gewaschen. Er ergaben sich 25,4 g (100 mmol) des gewünschten Produkts.
MS-FAB: 254 (M⁺ +1, 13)

### c) (S)-(2-Azido-1-methyl-ethyl)-carbaminsäure tert-butyl ester

Eine Lösung von 20,3 g (100 mmol) 1b in 155 ml DMSO wurden mit 7,8 g (120 mmol) Natriumazid versetzt und für 24 h bei 45°C gerührt. Es wurden 250 ml Eiswasser hinzugefügt. Die Mischung wurde mehrmalig mit 200 ml Dichlormethan extrahiert. Die vereinigten organischen Phasen wurden zweimal mit 50 ml wässriger, gesättigter Natriumchloridlösung gewaschen. Die organische Phase wurde eingeengt. Es ergaben sich 11,4 g Rohprodukt. Das Rohprodukt wurde ohne weitere Reinigung für die folgende Reaktion eingesetzt.
MS-FAB: 201 (M⁺+1, 23)

### d) (S)-(2-Amino-1-methyl-ethyl)-carbaminsäure tert-butyl ester

Eine gerührte Lösung von 11,4 g (57 mmol) 1c in 165 ml Essigsäureethylester wurden mit 1,8 g Pd/C (10%) versetzt und für 15 h einer Wasserstoffatmosphäre von 4 bar ausgesetzt. Der Katalysator wurde per Filtration (sogenannte G4-Fritte) abgetrennt. Das Filtrat wurde am Rotationsverdampfer eingeengt und säulenchromatographisch gereinigt (SiO₂ - Dichlormethan → Dichlormethan : Methanol 1:1). Es ergab sich das gewünschte Produkt in 73 % Ausbeute (7,25 g; 42 mmol).
MS-FAB: 175 (M⁺ +1, 29)

### e) (S,S)-{2-[2-tert-Butoxycarbonylamino-3-(4-nitro-phenyl)-propionylamino]-1-methyl-ethyl}-carbaminsäure tert-butyl ester

Eine gerührte Lösung von 7,2 g (41 mmol) 1d, 245 ml Wasser und 245 ml Dichlormethan wurden mit kommerziell erhältlichen 12,9 g (42 mmol) (S)-2-tert-Butoxycarbonylamino-3-(4-nitro-phenyl)-propionsäure *(Bachem)* und 6,4 g (42 mmol) 1-Hydroxybenzotriazol - H₂O (HOBT) versetzt. Die Lösung wurde auf 0° C gekühlt und mit 8,8 g (46 mmol) 1-(Dimethylaminopropyl)-3-ethylcarbodümid (EDCI) versetzt. Die Reaktionslösung wurde sieben Stunden bei 0° C, 12 Std. bei Raumtemperatur und 24 Stunden bei 60°C gerührt. Die Lösung wurde auf Raumtemperatur abgekühlt. Die wässrige Phase wurde abgetrennt und mehrmals mit Dichlormethan extrahiert. Die vereinigten organischen Phasen wurden mit 5%iger Natriumhydrogencarbonatlösung und wässriger, gesättigter Natriumchloridlösung gewaschen. Es wurde mit Natriumsulfat getrocknet und am Rotationsverdampfer eingeengt. Der Rückstand wurde mit Hexan versetzt, zerrieben, abgesaugt und mit kaltem Hexan nachgewaschen. Der Feststoff wurde im Vakuum bei 30° C getrocknet. Es ergab sich das gewünschte Produkt 1e mit 77% Ausbeute (14.9 g; 32 mmol).
MS-FAB: 467 (M⁺+1,38)

### f) (S,S)-N-(2-Amino-propyl)-3-(4-nitro-phenyl)-propan-1,2-diamin

14,9 g (32 mmol) 1e wurden in 180 ml Dichlormethan suspendiert. Anschließend wurden 54,2 g (475 mmol) Trifluoressigsäure hinzugetropft. Die Lösung wurde für eine Stunde gerührt und am Rotationsverdampfer eingeengt. Es wurde Dichlormethan hinzugefügt und nochmals eingeengt. Es wurde Diethylether hinzugefügt. Der ausfallende Feststoff wurde abgetrennt und mit kaltem Diethylether gewaschen. Der Feststoff wurde im Vakuum bei 35° C getrocknet. Zu einer gerührten Lösung dieses Feststoffes (17,7g) in 225 ml abs. THF wurden 225 ml (1 M) Boran-Tetrahydrofuran-Komplex hinzugetropft. Die Reaktionslösung wurde für 6 h unter Rückfluss erhitzt und anschließend über Nacht bei Raumtemperatur gerührt. Es wurden vorsichtig 60 ml Methanol hinzugetropft und es wurde für weitere 2 h gerührt. Die Lösung wurde am Rotationsverdampfer eingeengt und mit 150 ml Ethanol versetzt. Es wurde HCl-Gas eingeleitet, so dass ein Feststoff ausfiel. Es wurde eingeengt und mit trockenem Diethylether versetzt. Der Feststoff wurde abgetrennt, mit kaltem Diethylether gewaschen und im Vakuum bei 40° C getrocknet. Es ergaben sich 9,61 g (-26,6 mmol) des gewünschten Produktes 1f als Trihydrochlorid.
MS-FAB: 253 (M⁺ +1, 28)

### g) (S,S)-{[2-{[2-(Bis-tert-butoxycarbonylmethyl-amino)-propyl]-tert-butoxycarbonylmethyl-amino}-1-(4-nitro-benzyl)-ethyl]-tert-butoxycarbonylmethyl-amino-essigsäure tert-butyl ester

Zu einer gerührten Lösung von 9,6 g (27 mmol) 1f in 290 ml Acetonitril und 60 ml Wasser wurden 43,8 g (317 mmol) Kaliumcarbonat und 39 g (200 mmol) Bromessigsäure-tert-butylester hinzugegeben. Die Lösung wurde für ∼ 7h auf 70°C erwärmt. Es wurden weitere 13,1 g (94 mmol) Kaliumcarbonat und 8,8 ml (60 mmol) Bromessigsäure-tert-butylester hinzugegeben. Die Lösung wurde für ∼ 7h bei 70°C gerührt. Es wurden 4,3 g (26 mmol) Kaliumiodid hinzugegeben. Die Lösung wurde für ∼ 7h bei 70°C gerührt. Die Reaktionslösung wurde am Rotationsverdampfer eingeengt, mit Wasser versetzt und dreimal mit Essigester extrahiert. Die vereinigten organischen Phasen wurden mit wässriger, gesättigter Natriumchloridlösung gewaschen, mit Natriumsulfat getrocknet und eingeengt. Der Rückstand wurde säulenchromatographisch gereinigt (SiO₂ - Dichlormethan → Dichlormethan : Methanol 98:2). Es ergab sich das gewünschte Produkt 1g in 54 % Ausbeute (23,2 g; 42 mmol).
MS-FAB: 824 (M⁺ +1, 58)

### h) (S,S))-{[2-{[2-(Bis-carboxymethyl-amino)-propyl]-carboxymethyl-amino}-1-(4-nitro-benzyl)-ethyl]-carboxymethyl-amino}-essigsäure

Zu einer gerührten Lösung von 14,8 g (130 mmol) Trifluoressigsäure, 25,5 g (300 mmol) Dichlormethan und 2,9 g (25 mmol) Triethylsilian wurden 1,65g (2 mmol) 1g hinzugefügt. Die Reaktionsmischung wurde für 1 h gerührt und am Rotationsverdampfer und an der Ölpumpe eingeengt. Der Rückstand wurde mit Diethylether versetzt. Der Feststoff wurde durch Filtration abgetrennt und mit Diethylether gewaschen. Es ergab sich das gewünschte Produkt 1h mit 99% Ausbeute (1,0 g; 1,99 mmol).
MS-FAB: 543 (M⁺ +1, 59)

### Beispiel 2

### (S,S))-{[2-(4-Amino-phenyl)-1-({[2-(bis-tert-butoxycarbonylmethyl-amino)-propyl]-tert-butoxycarbonylmethyl-amino}-methyl)-ethyl]-tert-butoxycarbonylmethyl-amino}-essigsäure tert-butyl ester

Zu einer Lösung von 0,5 g (0,6 mmol) 1g in 10 ml iso-Propanol wurden 0,2 g Pd/C (10%) hinzugefügt. Die Atmosphäre über der Reaktionslösung wurde mit Wasserstoff versehen. Die Lösung wurde für 5 h gerührt, filtriert und eingeengt. Es ergab sich das gewünschte Produkt 2 in 81% (397 mg; 0,5 mmol).
MS-FAB: 795 (M⁺ +1, 63)

### Beispiel 3

### (S,S)-[(1-(4-Amino-benzyl)-2-{[2-(bis-carboxymethyl-amino)-propyl]-carboxymethyl-amino}-ethyl)-carboxymethyl-amino]-essigsäure

Methode A: Zu einer Lösung von 0,5 g (0,9 mmol) 1h in 10 ml iso-Propanol wurden 0,2 g Pd/C (10%) hinzugefügt. Die Atmosphäre über der Reaktionslösung wurde mit Wasserstoff versehen. Die Lösung wurde für 5 h gerührt, filtriert und eingeengt. Es ergab sich das gewünschte Produkt 3 in 87% (410 mg; 0,78 mmol).
MS-FAB: 513 (M⁺ +1, 43)

Methode B: Zu einer gerührten Lösung von 3,64 g (32 mmol) Trifluoressigsäure, 6,38 g (75 mmol) Dichlormethan und 0,7 g (6 mmol) Triethylsilian wurden 397 mg (0,5 mmol) 2 hinzugefügt. Die Reaktionsmischung wurde für 1 h gerührt und am Rotationsverdampfer und an der Ölpumpe eingeengt. Der Rückstand wurde mit Diethylether versetzt. Der Feststoff wurde durch Filtration abgetrennt und mit Diethylether gewaschen. Es ergab sich das gewünschte Produkt 3 mit 99% Ausbeute (253 mg; 0,5 mmol).
MS-FAB: 513 (M⁺ +1, 48)

### Beispiel 4

### (S,S)-[(2-{[2-(Bis-carboxymethyl-amino)-propyl]-carboxymethyl-amino}-1-{4-[3-(2,5-dioxo-2,5-dihydro-pyrrol-1-yl)-propionylamino]-benzyl}-ethyl)-carboxymethyl-amino]-essigsäure

Zu einer gerührten Lösung von 1.02 g (2 mmol) 3 und 774 mg (6 mmol) Diisopropylethylamin in 20 ml DMF wurden bei 0° C 800 mg (3 mmol) 3-(2,5-Dioxo-2,5-dihydro-pyrrol-1-yl)-propionsäure 2,5-dioxo-pyrrolidin-1-yl ester (Aldrich) hinzugefügt. Die Reaktionslösung wurde für 4 Stunden bei Raumtemperatur gerührt. Die Lösung wurde tropfenweise auf 120 ml heftig gerührten Diethylether gegeben. Die Suspension wurde für 30 Minuten gerührt und filtriert. Der Rückstand wurde an der Ölpumpe getrocknet. Ein Teil des Rückstands wurde durch semipräparative RP-HPLC gereinigt.
MS-FAB: 664 (M⁺ +1, 24)

### Beispiel 5

### (S,S)-[(1-(4-Isothiocyanato-benzyl)-2-{[2-(bis-carboxymethyl-amino)-propyl]-carboxymethyl-amino}-ethyl)-carboxymethyl-amino]-essigsäure

Zu einem heftig gerührten Zwei-Phasen-System aus 0,51 g (1 mmol) 3, 3 ml Wasser, 605 mg (6 mmol) Triethylamin und 3 ml Chloroform wurden bei 0° C 114 mg (1 mmol) Thiophosgen in wenig Chloroform hinzugetropft. Die Lösung wurde für 3 Stunden gerührt. Die organische Phase wurde abgetrennt. Die organische Phase wurde zweimal mit Wasser extrahiert. Die vereinigten, wässrigen Phasen wurden mit Dichlormethan gewaschen, mit Wasser verdünnt und gefriergetrocknet. Die Substanz wurde per HPLC auf Reinheit überprüft: HyPurity C18 (5□m, 150 x 3,0 mm) mit Acetonitril- : Wasser- : Trifluoressigsäure-Gradient (3 : 96,9 : 0,1 → 99,9 : 0 : 0,1). Es ergab sich das gewünschte Produkt 5 mit 91 % Ausbeute (505 mg, 910 mmol).
MS-FAB: 555 (M⁺ +1,39)

### Beispiel 6

### (S,S)-({2-{[2-(Bis-carboxymethyl-amino)-propyl]-carboxymethyl-amino}-1-[4-(2-bromo-acetylamino)-benzyl]-ethyl}-carboxymethyl-amino)-essigsäure

Zu einer Lösung von 0,51 g (1 mmol) 3 und 606 mg (6 mmol) Triethylamin in 10 ml DMF wurden bei -20°C 202 mg (1,1 mmol) Bromessigsäurebromid hinzugefügt. Die Reaktionslösung wurde für eine Stunden bei Raumtemperatur gerührt. Die Lösung wurde auf heftig gerührten Diethylether gegossen. Der Niederschlag wurde abfiltriert, in Wasser aufgenommen und unverzüglich gefriergetrocknet. Die Substanz wurde per HPLC auf Reinheit überprüft: HyPurity C18 (5µm, 150 x 3,0 mm) mit Acetonitril- : Wasser- : Trifluoressigsäure-Gradient (3 : 96,9 : 0,1 → 99,9 : 0 : 0,1). Es ergab sich das gewünschte Produkt 6 mit 82 % Ausbeute (520 mg, 820 µmol).
MS-FAB: 634 (M⁺ +1, 46)

### Beispiel 7

### (S,S)-({2-{[2-(Bis-carboxymethyl-amino)-propyl]-carboxymethyl-amino}-1-[4-(2-iodo-acetylamino)-benzyl]-ethyl}-carboxymethyl-amino)-essigsäure

Zu einer Lösung von 0,51 g (1 mmol) 3 und 606 mg (6 mmol) Triethylamin in 10 ml DMF wurden bei -20°C 274 mg (1,1 mmol) lodessigsäurebromid hinzugefügt. Die Reaktionslösung wurde für eine Stunden bei Raumtemperatur gerührt. Die Lösung wurde auf heftig gerührten Diethylether gegossen. Der Niederschlag wurde abfiltriert, in Wasser aufgenommen und unverzüglich gefriergetrocknet. Die Substanz wurde per HPLC auf Reinheit überprüft: HyPurity C18 (5µm, 150 x 3,0 mm) mit Acetonitril- : Wasser- : Trifluoressigsäure-Gradient (3 : 96,9 : 0,1 → 99,9 : 0 : 0,1). Es ergab sich das gewünschte Produkt 7 mit 88 % Ausbeute (600 mg, 880 µmol).
MS-FAB: 681 (M⁺+1,32)

### Beispiel 8

### (S,S)-({2-{[2-(Bis-carboxymethyl-amino)-propyl]-carboxymethyl-amino}-1-[4-(2-iodo-acetylamino)-benzyl]-ethyl}-carboxymethyl-amino)-essigsäure

Zu einer Lösung von 0,51 g (1 mmol) 3 und 606 mg (6 mmol) Triethylamin in 10 ml DMF wurden bei -20°C 125 mg (1,1 mmol) Chloressigsäurechlorid hinzugefügt. Die Reaktionslösung wurde für eine Stunden bei Raumtemperatur gerührt. Die Lösung wurde auf heftig gerührten Diethylether gegossen. Der Niederschlag wurde abfiltriert, in Wasser aufgenommen und unverzüglich gefriergetrocknet. Die Substanz wurde per HPLC auf Reinheit überprüft: HyPurity C18 (5µm, 150 x 3,0 mm) mit Acetonitril- : Wasser- : Trifluoressigsäure-Gradient (3 : 96,9 : 0,1 → 99,9 : 0 : 0,1). Es ergab sich das gewünschte Produkt 8 mit 82 % Ausbeute (520 mg, 820 µmol).
MS-FAB: 590 (M⁺ +1, 31)

### Beispiel 9

### (S,S)-({2-{[2-(Bis-carboxymethyl-amino)-propyl]-carboxymethyl-amino}-1-[4-(2,5-dioxo-2,5-dihydro-pyrrol-1-yl)-benzyl]-ethyl}-carboxymethyl-amino)-essigsäure

In Anlehnung an *Tetrahedron Lett*.; 38; 46; 1997; 8089-8092:
Ein Gemisch aus ca. 1 g getrocknetem Kieselgel, 100 mg (1,0 mmol) Maleinsäureanhydrid, 512 mg (1,0 mmol) 3 und 35,6 mg (0,1 mmol) Tantal-(V)-chlorid wurden in der Mikrowelle (300W) für 5 min erhitzt. Der Rückstand wurde über einer Fritte mit Methanol eluiert. Das Filtrat wurde eingeengt. Der Rückstand wurde in Wasser aufgenommen. Die Lösung wurde gefriergetrocknet. Ein Teil des Rückstands wurde durch semipräparative RP-HPLC gereinigt. Acetonitril-Wasser-Gemisch (20:80). Die Substanz wurde per HPLC auf Reinheit überprüft: HyPurity C18 (5□m, 150 x 3,0 mm) mit Acetonitril- : Wasser- : Trifluoressigsäure-Gradient (3 : 96,9 : 0,1 → 99,9 : 0 : 0,1). Es ergab sich das gewünschte Produkt 9 mit 94 mg (0,16 mmol) in 96 % Reinheit.
MS-FAB: 593 (M⁺ +1, 42)

### Beispiel 10

### Antikörperkonjugat von Beispiel 4 nämlich (S,S)-[(2-{[2-(Bis-carboxymethyl-amino)-propyl]-carboxymethyl-amino}-1-{4-[3-(2,5-dioxo-2,5-dihydro-pyrrol-1-yl)-propionylamino]-benzyl}-ethyl)-carboxymethyl-amino]-essigsäure

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 159 µg (240 nmol) Produkt aus Beispiel 4, gelöst in 50 µl Boratpuffer (s.o.), versetzt und 3 Stunden bei 37°C gerührt. Es wurde über eine NAP-5-Säule (Amersham Pharmacia Biotech AB, Sephadex G-25, Mobile Phase: PBS) gereinigt.

### Beispiel 11

### (S,S)-{[2-{[2-(Bis-carboxymethyl-amino)-propyl]-carboxymethyl-amino}-1-(4-{3-[2-(2,5-dioxo-2,5-dihydro-pyrrol-1-yl)-ethyl]-thioureido}-benzyl)-ethyl]-carboxymethyl-amino}-essigsäure

Zu einer gerührten Lösung von 555 mg (1 mmol) 5 in 5 ml DMF wurde eine Lösung von 154 mg (1,1 mmol) 1-(2-Amino-ethyl)-pyrrol-2,5-dion in 2 ml Dioxan hinzugetropft. Die Reaktionslösung wurde über Nacht gerührt und zu 80 ml heftig gerührten Diethylether tropfenweise hinzugegeben. Der Niederschlag wurde abfiltriert und in Wasser aufgenommen. Die Lösung wurde gefriergetrocknet. Ein Teil des Rückstands wurde durch semipräparative RP-HPLC gereinigt. Acetonitril-Wasser-Gemisch (20:80). Die Substanz wurde per HPLC auf Reinheit überprüft: HyPurity C18 (5µm, 150 x 3,0 mm) mit Acetonitril- : Wasser- : Trifluoressigsäure-Gradient (3 : 96,9 : 0,1 → 99,9 : 0 : 0,1). Es ergab sich das gewünschte Produkt 11 mit 0,104 g (0,15 mmol) in hoher Reinheit.
MS-FAB: 696 (M⁺ +1, 33)

### Beispiel 12

### Indiumkomplex des Antikörperkonjugats von von Beispiel 4 nämlich (S,S)-[(2-{[2-(Bis-carboxymethyl-amino)-propyl]-carboxymethyl-amino}-1-{4-[3-(2,5-dioxo-2,5-dihydro-pyrrol-1-yl)-propionylamino]-benzyl}-ethyl)-carboxymethyl-amino]-essigsäure

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 159 µg (240 nmol) Produkt aus Beispiel 11, gelöst in 50 µl Boratpuffer (s.o.), versetzt und 3 Stunden bei 37°C gerührt. Die Borat-Pufferlösung wurde gegen einen Acetatpuffer ausgetauscht, indem die Probelösung dreimal für 1h im Slide-A-Lyzer 10000, Pierce MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1 M (pH 6) gestellt. Die Lösung wurde mit 80 µl (0.05M HCl) [¹¹¹ln]lnCl₃ (27,88 MBq) versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 13

### Yttriumkomplex des Antikörperkonjugats von (1'R,2R,5S)-5-({[2-({1-Carboxy-5-[3-(2,5-dioxo-2,5-dihydro-pyrrol-1-yl)-propionylamino]-pentyl}-carboxymethyl-amino)-ethyl]-carboxymethyl-amino}-methyl)-1-carboxymethyl-pyrrolidin-2-carbonsäure

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 159 µg (240 nmol) Produkt aus Beispiel 11, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq f⁹⁰Y]YCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 14

### Scandiumkomplex des Antikörperkonjugats von (1'R,2R,5S)-5-({[2-({1-Carboxy-5-[3-(2,5-dioxo-2,5-dihydro-pyrrol-1-yl)-propionylamino]-pentyl}-carboxymethyl-amino)-ethyl]-carboxymethyl-amino}-methyl)-1-carboxymethyl-pyrrolidin-2-carbonsäure

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 159 µg (240 nmol) Produkt aus Beispiel 11, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq [⁴⁷Sc]ScCl3 versetzt und für 30 min bei Raumtemperatur gerührt. Es wurde über eine NAP-5-Säule (Amersham Pharmacia Biotech AB, Sephadex G-25, Mobile Phase: PBS)

### gereinigt.

### Beispiel 15

### Lutetiumkomplex des Antikörperkonjugats von (1'R,2R,5S)-5-({[2-({1-Carboxy-5-[3-(2,5-dioxo-2,5-dihydro-pyrrol-1-yl)-propionylamino]-pentyl}-carboxymethyl-amino)-ethyl]-carboxymethyl-amino}-methyl)-1-carboxymethyl-pyrrolidin-2-carbonsäure

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 159 µg (240 nmol) Produkt aus Beispiel 11, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq [¹⁷⁷Lu]LuCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 16

### a) (S)-6-Benzyloxycarbonylamino-2-(2-tert-butoxycarbonylamino-acetylamino)-hexansäure benzyl ester

Zu einer gerührten Lösung von 2,15 g (12,28 mmol) Boc-Gly-OH und 4,08 ml (29,5 mmol) Triethylamin in 50 ml Dichlormethan wurden 1,55 g (13,5 mmol) N-Hydroxysuccinimid hinzugefügt. Nach 20 min wurden 5 g (12,3 mmol) H-Lys-(Z)-OBzl Hydrochlorid, welches in etwas Dichlormethan gelöst wurde, und 2,78 g (13,5 mmol) Dicyclohexylcarbodümid in etwas Dichlormethan hinzugefügt. Die Lösung wurde für drei Tage gerührt und auf 250 ml Eiswasser gegossen. Die organische Phase wurde abgetrennt. Die wässrige Phase wurde mehrmals mit Dichlormethan extrahiert. Die vereinigten organischen Phasen wurden mit wässriger, gesättigter Natriumchloridlösung gewaschen, mit Natriumsulfat getrocknet und eingeengt. Der Rückstand wurde säulenchromatographisch gereinigt (SiO₂ Hexan: Essigester 4:1 → Hexan: Essigester 1:1). Es ergab sich das gewünschte Produkt 16a in 96% Ausbeute (6,2 g; 11,7 mmol).
MS-FAB: 528 (M⁺ +1, 75)

### b) (S)-6-Benzyloxycarbonylamino-2-(2-{2-[2-(2-tert-butoxycarbonylamino-acetylamino)-acetylamino]-acetylamino}-acetylamino)-hexansäure benzyl ester

Zu einer Lösung von 6,2 g (11,7 mmol) 16a in 30 ml Dichlormethan wurden bei 0°C 30 ml Trifluoressigsäure tropfenweise hinzugefügt. Die Lösung wurde für 2 h bei Raumtemperatur gerührt und am Rotationsverdampfer eingeengt. Es wurden 50 ml Wasser und 50 ml Toluol hinzugefügt und wieder am Rotationsverdampfer entfernt. Der letzte Arbeitsschritt wurde dreimal wiederholt. Abschließend wurde an der Ölpumpe konzentriert.
Eine gerührte Lösung des Rückstands in 90 ml Dichlormethan wurden bei 0°C mit 2,37 g (18,3 mmol) Diisopropylethyldiamin, 3,02 g (14,7 mmol) Dicyclohexylcarbodiimid, gelöst in wenig Dichlormethan, 1,69 g (14,7 mmol) N-Hydroxysuccinimid und 3,4 g (14,7 mmol) Boc-Gly-Gly-OH hinzugefügt. Die Suspension wurde drei Tage bei Raumtemperatur gerührt und mit 150 ml Eiswasser versetzt. Die organische Phase wurde abgetrennt. Die wässrige Phase wurde mehrmals mit Essigester extrahiert. Die vereinigten organischen Phasen wurden mit wässriger, gesättigter Natriumchloridlösung gewaschen, mit Natriumsulfat getrocknet und eingeengt. Der Rückstand wurde säulenchromatographisch gereinigt (SiO₂ Essigester → Methanol : Essigester 15:85). Es ergab sich das gewünschte Produkt 16b in 53% Ausbeute (4,18 g; 6,5 mmol).
MS-FAB: 643 (M⁺ +1, 56)

### c) (S)-2-{2-[2-(2-tert-Butoxycarbonylamino-acetylamino)-acetylamino]-acetylamino}-6-[3-(2,5-dioxo-2,5-dihydro-pyrrol-1-yl)-propionylamino]-hexansäure

Zu einer Lösung von 4,18 g (6,5 mmol) 16b in 40 ml Isopropanol wurden 1 g Pd/C (10%) hinzugefügt. Die Atmosphäre über der gerührten Lösung wurde mit Wasserstoff gesättigt. Die Reaktionslösung wurde für 90 min gerührt, filtriert und eingeengt.
Der Rückstand wurde bei 0° C mit 20 ml DMF, 1,5 g (12 mmol) Diisopropylethylamin und 2,4 mg (9 mmol) 3-(2,5-Dioxo-2,5-dihydro-pyrrol-1-yl)-propionsäure 2,5-dioxo-pyrrolidin-1-yl ester (Aldrich) versetzt. Die Reaktionslösung wurde für 4 Stunden bei Raumtemperatur gerührt. Die Lösung wurde in HCl-Lösung (pH 4) gegeben und mit Essigester mehrmals extrahiert. Die vereinigten organischen Phasen wurden mit wässriger, gesättigter Natriumchloridlösung gewaschen, mit Natriumsulfat getrocknet und eingeengt. Der Rückstand wurde säulenchromatographisch gereinigt (SiO₂ Essigester → Methanol : Essigester 20:80). Es ergab sich das gewünschte Produkt 16c in 67% Ausbeute (2,5 g; 4,4 mmol).
MS-FAB: 570 (M⁺ +1, 31)

### d) (1S,1'S,4S)-1-[4-{3-[({[({1-Carboxy-5-[3-(2,5-dioxo-2,5-dihydro-pyrrol-1-yl)-propionylamino]-pentylcarbamoyl}-methyl)-carbamoyl}-methyl}-carbamoyl)-methyl]-thioureido}]-benzyl-4-methyl-DTPA

Zu einer gerührten Lösung von 570 mg (1 mmol) 16c in 5 ml Dichlormethan wurden bei 0°C 5ml Trifluoressigsäure tropfenweise hinzugefügt. Die Lösung wurde für 2h bei Raumtemperatur gerührt und am Rotationsverdampfer eingeengt. Es wurde der Rückstand mit Diethylether ausgerührt. Abschließend wurde an der Ölpumpe konzentriert.
Der Rückstand wurde in DMF aufgenommen mit - 200 mg (~2 mmol) Triethylamin und 505 mg (0,9 mmol) 5 versetzt. Die Lösung wurde für 3 Std. bei 40°C gerührt und auf heftig gerührten Diethylether getropft. Der Niederschlag wurde abfiltriert und RP-säulenchromatographisch gereinigt.
MS-FAB: 1016 (M⁺ +1, 31)

### Beispiel 17

### Gadolinium-Komplex von (S,S)-[(1-(4-Amino-benzyl)-2-{[2-(bis-carboxymethyl-amino)-propyl]-carboxymethyl-amino}-ethyl)-carboxymethyl-amino]-essigsäure

128,1 mg (0,25 mmol) 3 wurden in 4 ml destilliertem Wasser suspendiert, auf 80°C erwärmt und in Lösung gebracht. Es wurde portionsweise mit 45,3 mg (0,125 mmol) Gd₂O₃ versetzt. Die Suspension wurde auf 80° C erwärmt und für eine Stunde gerührt. Die Lösung wurde auf Raumtemperatur abgekühlt und mit Natronlauge (1M) auf pH=7 eingestellt. Es wurde das Wasser durch Gefriertrocknung entfernt. Es ergab sich das gewünschte Produkt mit 166,6 mg (0,25 mmol, 99,8%).
MS-FAB (M⁺ +1, 21): 668

### Beispiel 18

### Antikörperkonjugat von (1S,1'S,4S)-1-[4-{3-[({[({1-Carboxy-5-[3-(2,5-dioxo-2,5-dihydro-pyrrol-1 -yl)-propionylamino]-pentylcarbamoyl}-methyl)-carbamoyl]-methyl}-carbamoyl)-methyl]-thioureido}]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 243 µg (240 nmol) Produkt aus Beispiel 16d, gelöst in 50 µl Boratpuffer (s.o.), versetzt und 3 Stunden bei 37°C gerührt. Es wurde über eine NAP-5-Säule (Amersham Pharmacia Biotech AB, Sephadex G-25, Mobile Phase: PBS) gereinigt.

### Beispiel 19

### Yttriumkomplex des Antikörperkonjugats von (1S,1'S,4S)-1-[4-{3-[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-thioureido}]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 243 µg (240 nmol) Produkt aus Beispiel 16d, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq [⁹⁰Y]YCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 20

### Scandiumkomplex des Antikörperkonjugats (1S,1'S,4S)-1-[4-{3-[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-thioureido}]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 243 µg (240 nmol) Produkt aus Beispiel 16d, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq [⁴⁷Sc]ScCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 21

### Lutetiumkomplex des Antikörperkonjugats (1S,1'S,4S)-1-[4-(3-[({[({1-' Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-thioureido}]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 243 µg (240 nmol) Produkt aus Beispiel 16d, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1 M (pH 6) gestellt. Die Lösung wurde mit 50 MBq [¹⁷⁷Lu]LuCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 22

### (R,R)-{[2-{[2-(Bis-carboxymethyl-amino)-propyl]-carboxymethyl-amino}-1-(4-nitro-benzyl)-ethyl]-carboxymethyl-amino}-essigsäure

Die Synthese von Beispiel 22 wird analog zu Beispiel 1 vorgenommen, mit dem Unterschied, dass nicht (S)- sondern (R)-2-Amino-1-propanol eingesetzt, und auch nicht (S)- sondern (R)-2-tert-Butoxycarbonylamino-3-(4-nitro-phenyl)-propionsäure als Baustein verwandt wurde. Es ergab sich das gewünschte Produkt 22 in hoher Reinheit (>96%, RP-HPLC)
MS-FAB: 513 (M⁺+1, 42)

### Beispiel 23

### (R,R))-[(1-(4-Amino-benzyl)-2-{[2-(bis-carboxymethyl-amino)-propyl]-carboxymethyl-amino}-ethyl)-carboxymethyl-amino]-essigsäure

Die Synthese von Beispiel 23 wird analog zu Beispiel 3 Methode A vorgenommen, mit dem Unterschied, dass als Edukt nicht 1h, sondern 22 eingesetzt wurde. Es ergab sich das gewünschte Produkt 23 mit 96% Ausbeute.
MS-FAB: 513 (M⁺ +1, 42)

### Beispiel 24

### (R,R))-{[2-(4-Amino-phenyl)-1-({[2-(bis-tert-butoxycarbonylmethyl-amino)-propyl]-tert-butoxycarbonylmethyl-amino}-methyl)-ethyl]-tert-butoxycarbonylmethyl-amino}-essigsäure tert-butyl ester

Die Synthese von Beispiel 24 wird analog zu Beispiel 2 vorgenommen, mit dem Unterschied, dass als Edukt nicht 1g, sondern die entsprechende Vorstufe aus der Synthese von 22 eingesetzt wurde. Es ergab sich das gewünschte Produkt 24 mit 86% Ausbeute.
MS-FAB: 794 (M⁺ +1, 53)

### Beispiel 25

### (R,R)-[(1-(4-Isothiocyanato-benzyl)-2-{[2-(bis-carboxymethyl-amino)-propyl]-carboxymethyl-amino}-ethyl)-carboxymethyl-amino]-essigsäure

Die Synthese von Beispiel 25 wird analog zu Beispiel 5 vorgenommen, mit dem Unterschied, dass als Edukt nicht 3, sondern 23 eingesetzt wurde. Es ergab sich das gewünschte Produkt 25 mit 74% Ausbeute.
MS-FAB: 555 (M⁺ +1, 33)

### Beispiel 26

### (R,R)-({2-{[2-(Bis-carboxymethyl-amino)-propyl]-carboxymethyl-amino}-1-[4-(2-bromo-acetylamino)-benzyl]-ethyl}-carboxymethyl-amino)-essigsäure

Die Synthese von Beispiel 26 wird analog zu Beispiel 6 vorgenommen, mit dem Unterschied, dass als Edukt nicht 3, sondern 23 eingesetzt wurde. Es ergab sich das gewünschte Produkt 23 mit 71 % Ausbeute.
MS-FAB: 590 (M⁺ +1, 48)

### Beispiel 27

### a) N-[4-(2-(Bis-carboxymethyl-amino)-3-{[2-(bis-carboxymethyl-amino)-propyl]-carboxymethyl-amino}-propyl)-phenyl]-succinamin säure

Zu einer Lösung von 793 mg (1 mmol) 24 und 0,28 ml (-2 mmol) Disopropylethylamin in 20 ml THF wurden 200 mg (2 mmol) Bernsteinsäureanhydrid hinzugefügt. Die Lösung wurde für 2h bei Raumtemperatur gerührt. Die Lösung wurde auf ein Drittel des Volumens eingeengt mit Dichlormethan verdünnt und auf eine wässrige Lösung (pH=4,5) gegeben. Die wässrige Phase wurde abgetrennt und mehrmals mit Dichlormethan extrahiert. Die vereinigten organischen Phasen wurden mit Natriumchloridlösung gewaschen. Es wurde mit Natriumsulfat getrocknet und am Rotationsverdampfer eingeengt. Der Rückstand wurde säulenchromatographisch gereinigt (CH₂Cl₂ - MeOH). Es ergab sich das gewünschte Produkt 27a mit 85% Ausbeute (0,85 mmol).
MS-FAB: 613 (M⁺ +1, 58)

### b) (1R,1'S,4R)-1-[4-(3-{[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-carbamoyl}-propionyl)]-benzyl-4-methyl-DTPA

Zu einer gerührten Lösung von 570 mg (1 mmol) 16c in 5 ml Dichlormethan wurden bei 0°C 5ml Trifluoressigsäure tropfenweise hinzugefügt. Die Lösung wurde für 2h bei Raumtemperatur gerührt und am Rotationsverdampfer eingeengt. Es wurde der Rückstand mit Diethylether ausgerührt. Abschließend wurde an der Ölpumpe konzentriert. Der Rückstand wurde in Dichlormethan suspendiert, mit 0,25 g (-2 mmol) Hünigbase, 304 mg (2 mmol) 1-Hydroxybenzotriazol - H₂O (HOBT) und 122 mg (2 mmol) 27a versetzt. Die Lösung wurde auf 0° C gekühlt und mit 419 mg (21 mmol) 1-(Dimethylaminopropyl)-3-ethylcarbodiimid (EDCI) versetzt. Die Lösung wurde für 12 Std. gerührt und auf Eiswasser gegossen. Die wässrige Phase wurde abgetrennt und mehrmals mit Dichlormethan extrahiert. Die vereinigten organischen Phasen wurden mit Natriumchloridlösung gewaschen. Es wurde mit Natriumsulfat getrocknet und am Rotationsverdampfer eingeengt. Der Rückstand wurde säulenchromatographisch gereinigt (CH₂Cl₂ - Acetonitril). Es ergab sich das gewünschte Produkt mit 85% Ausbeute (0,85 mmol), welches dann in 5 ml Dichlormethan und 3 ml Anisol aufgenommen wurde und mit 5 ml Trifluoressigsäure bei 0°C versetzt wurde. Die Lösung wurde für 8 Std. gerührt, eingeengt und mit Diethylether ausgerührt. Es ergab sich das gewünschte Produkt 27b in 90% Ausbeute (813,9 mg).
MS-FAB: 1064 (M⁺ +1,38)

### Beispiel 28

### Antikörperkonjugat von (1R,1'S,4R)-1-[4-(3-{[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-carbamoyl}-propionyl)]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 255 µg (240 nmol) Produkt aus Beispiel 27b, gelöst in 50 µl Boratpuffer (s.o.), versetzt und 3 Stunden bei 37°C gerührt. Es wurde über eine NAP-5-Säule (Amersham Pharmacia Biotech AB, Sephadex G-25, Mobile Phase: PBS) gereinigt.

### Beispiel 29

### Yttriumkomplex des Antikörperkonjugats von (1R,1'S,4R)-1-[4-(3-{[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-carbamoyl}-propionyl)]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 255 µg (240 nmol) Produkt aus Beispiel 27b, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1 M (pH 6) gestellt. Die Lösung wurde mit 50 MBq [⁹⁰Y]YCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 30

### Lutetiumkomplex des Antikörperkonjugats von (1R,1'S,4R)-1-[4-(3-{[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-carbamoyl}-propionyl)]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 255 µg (240 nmol) Produkt aus Beispiel 27b, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq [¹⁷⁷Lu]LuCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

### Beispiel 31

### Scandiumkomplex des Antikörperkonjugats von (1R,1'S,4R)-1-[4-(3-{[({[({1-Carboxy-5-[3-(2,5-dioxo-2,5-dihydro-pyrrol-1-yl)-propionylamino]-pentylcarbamoyl}-methyl)-carbamoyl]-methyl}-carbamoyl)-methyl]-carbamoyl}-propionyl)]-benzyl-4-methyl-DTPA

200 µg eines Antikörpers mit freizugänglichen Thiolgruppen (z.B. HuM195 (vgl. Michael R. McDevitt, J. Nuc. Med. 40, 1999, 1722; im Handel erhältlich bei Protein Design Labs Inc., Mountainview, CA, USA) - besitzt der Antikörper keine frei zugänglichen Thiolgruppen, können diese durch die Verwendung von 2-Iminothiolan HCl erzeugt werden (z.B. EP 0 607 222 B1)) wurden in 1,2 ml Boratpuffer (50 mM, pH 8,5) verdünnt, mit 255 µg (240 nmol) Produkt aus Beispiel 27b, gelöst in 50 µl Boratpuffer (s.o.), versetzt und für 3 Stunden bei 37° C gerührt. Die Borat-Pufferlösung wurde gegen einen Acetatpuffer ausgetauscht, indem die Probelösung dreimal für 1h im Slide-A-Lyzer 10000, Pierce, MWCO (Dialyse-Verfahren) gegen jeweils 200 ml NaOAc-Puffer 0,1 M (pH 6,0) gestellt wurde. Abschließend wurde über Nacht gegen 400 ml NaOAc-Puffer 0,1M (pH 6) gestellt. Die Lösung wurde mit 50 MBq [⁴⁷Sc]ScCl₃ versetzt und für 30 min bei Raumtemperatur gerührt. Es wurde über eine NAP-5-Säule (Amersham Pharmacia Biotech AB, Sephadex G-25, Mobile Phase: PBS) gereinigt.

## Patentansprüche

1. Verbindungen der allgemeinen Formel VIIa und VIIb 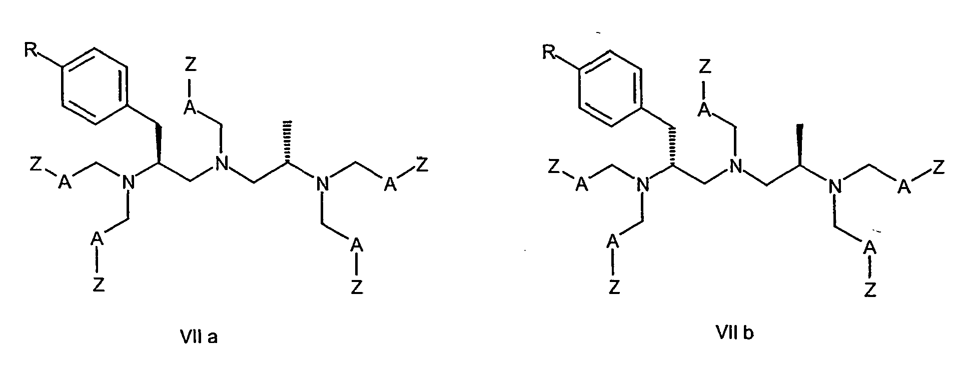 worin
Z für ein Wasserstoffatom oder ein Metallionenäquivalent eines Elements der Ordnungszahl 21-29, 31, 32, 37-39, 42-44, 46, 47, 49, 58-71, 75, 77, 82 oder 83 steht,
A für eine Gruppe -COO- steht,
R für eine Nitrogruppe, eine Aminogruppe, oder eine andere funktionelle Gruppe, die mit einem Biomolekül verknüpft werden kann, oder für einen geradkettigen oder verzweigten, gesättigten oder ungesättigten und gegebenenfalls durch ein bis sechs O-Atome, bzw. Phenylen, -NHCO-, -CONH-, 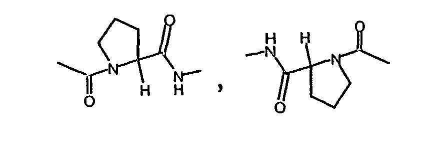 und/oder -NH-(C=S)-NH-Gruppen unterbrochenen C₁ - C₂₅ -Alkylrest, der gegebenenfalls an einer beliebigen Stelle mit ein bis sechs Carboxylgruppen, Hydroxylgruppen, Aminogruppen oder anderen funktionellen Gruppen substituiert ist, sowie deren Salze mit organischen oder anorganischen Basen mit den Maßgaben, dass der Alkylrest mindestens eine funktionelle Gruppe, die mit einem Biomolekül verknüpft werden kann, enthält und dass mindestens zwei Z für ein Metallionenäquivalent stehen.

2. Verbindungen nach Anspruch 1, worin mindestens zwei der Reste Z für ein Metallionenäquivalent eines paramagnetischen Elements der Ordnungszahlen 21-29, 42, 44 und 58-70 stehen.

3. Verbindungen nach Anspruch 1, worin mindestens zwei der Reste Z für ein Metallionenäquivalent eines radioaktiven Elements der Ordnungszahlen 26, 27, 29, 31, 32, 37-39, 43, 46, 47, 49, 61, 62, 64, 70, 71, 75, 77, 82 und 83 stehen.

4. Verbindungen nach Anspruch 1, worin R für einen Rest 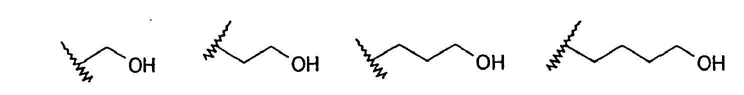 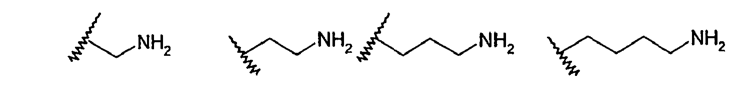 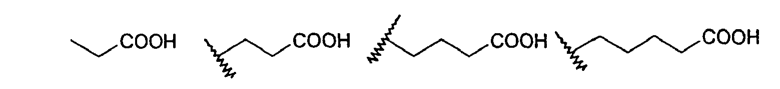 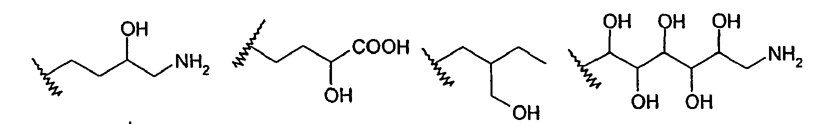 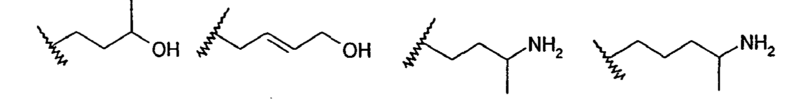 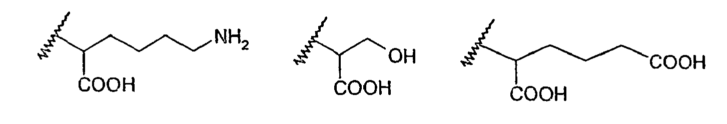 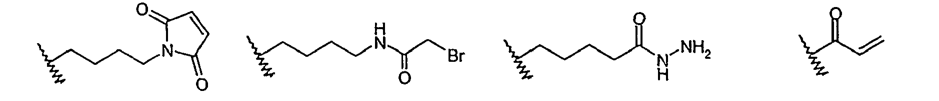 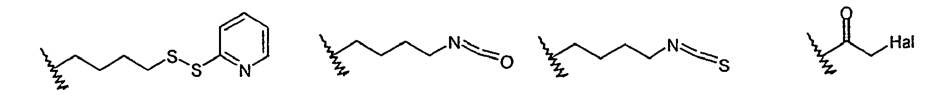 steht.

5. Verbindungen nach Anspruch 1, worin R für einen Rest 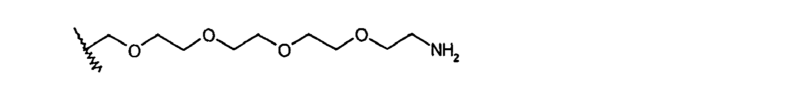 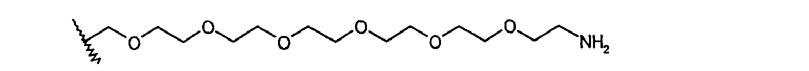 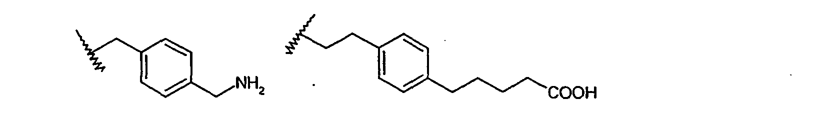 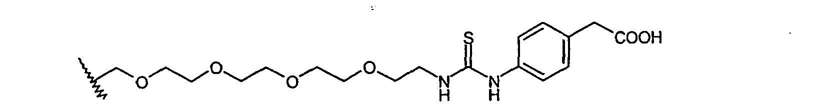 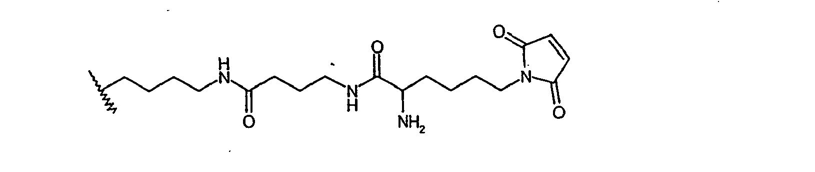 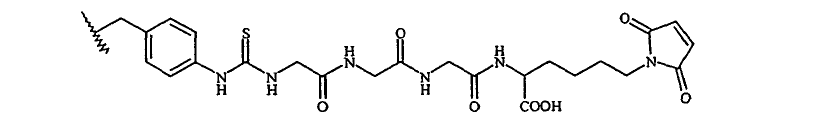 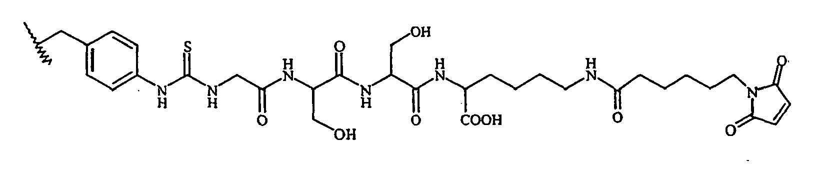 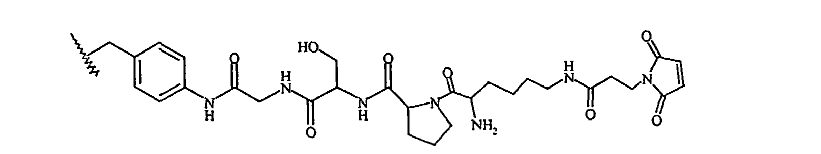 steht.

6. Verbindungen nach Anspruch 1, worin R für eine funktionelle Gruppe Carboxyl, aktiviertes Carboxyl, Amino, Nitro, Isocyanat, Isothiocyanat, Hydrazin, Semicarbazid, Thiosemibarbazid, Chloracetamid, Bromacetamid, lodacetamid, Acryl, Acylamino, gemischten Anhydriden, Azid, Säurechlorid, Säurebromid, Hydroxid, Sulfonylchlorid, Vinylsulphon, Carbodiimid, Maleimid oder Diazo steht.

7. Verbindungen nach Anspruch 6, worin die aktivierte Carboxylgruppe ausgewählt ist aus 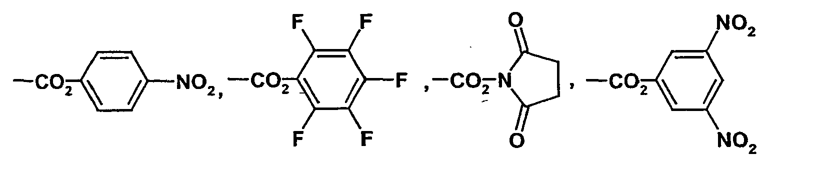 und

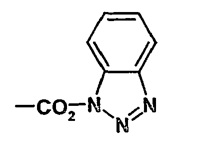

8. Verwendung von Verbindungen der allgemeinen Formel VIIa oder VIIb nach Anspruch 1 zur Herstellung von Konjugaten mit einem Biomolekül.

9. Verwendung nach Anspruch 8, worin das Biomolekül ausgewählt ist aus der Gruppe bestehend aus:
Biopolymere, Proteine, wie Proteine, die eine biologische Funktion haben, HSA, BSA, etc., Proteine und Peptide, die sich an bestimmten Stellen im Organismus anreichern (z.B. an Rezeptoren, Zellmembranen, Kanälen etc.), durch Proteasen spaltbare Peptide, Peptide mit synthetischen Sollbruchstellen (z.B. labile Ester, Amide etc.), Peptide, die durch Metalloprotheasen gespalten werden, Peptide mit photospaltbaren Linkern, Peptide mit oxydativen Mitteln (Oxydasen) spaltbaren Gruppen, Peptide mit natürlichen und unnatürlichen Aminosäuren, Glycoproteine (Glycopeptide), Signal-Proteine, antivirale Proteine und Apoktosis, synthetisch modifizierte Biopolymere, wie mit Linkern derivatisierte Biopolymere, modifizierte Metalloproteasen und derivatisierte Oxydase etc., Kohlenhydrate (Mono- bis Polysaccharide), wie derivatisierte Zucker, im Organismus spaltbare Zucker, Cyclodextrine und dessen Derivate, Aminozucker, Chitosan, Polysulfate und Acetylneuraminsäure-Derivate, Antikörper, wie monoklonale Antikörper, Antikörperfragmente, polyklonale Antikörper, Minibodies, Single Chains (auch solche, die mit Linkern zu mehrfachen Fragmenten verknüpft sind), rote Blutkörperchen und andere Blutbestandteile, Cancermarker (z.B. CAA) und Zell-Adhäsions-Stoffe (z.B. Lewis X und Anti-Lewis X-Derivate), DNA und RNA Fragmente, wie derivatisierte DNAs und RNAs (z.B. solche, die durch das SELEX-Verfahren gefunden wurden), synthetische RNA und DNA (auch mit unnatürlichen Basen), PNAs (Hoechst) und Antisense, β-Aminosäuren (Seebach), Vektoramine zur Einschleusung in die Zelle, biogene Amine, Pharmazeutika, onkologische Präparate, synthetische Polymere, die auf ein biologisches Target (z.B. Rezeptor) gerichtet sind, Steroide (natürliche und modifizierte), Prostaglandine, Taxol und dessen Derivate, Endotheline, Alkaloide, Folsäure und deren Derivate, bioaktive Lipide, Fette, Fettsäureester, synthetisch modifizierte Mono-, Di- und Triglyceride, Liposome, die an der Oberfläche derivatisiert sind, Micellen aus natürlichen Fettsäuren oder aus Perfluoralkyl-Verbindungen, Porphyrine, Texaphrine, erweitere Porphyrine, Cytochrome, Inhibitoren, Neuramidasen, Neuropeptide, Immunomodulatoren, wie FK 506, CAPE und Gliotoxin, Endoglycosidasen, Substrate, die durch Enzyme aktiviert werden wie Calmodolin Kinase, Casein-Kinase II, Gluthathion-S-Transferase, Heparinase, Matrix-Metalloprotheasen, β-Insulin-Rezeptor-Kinase, UDP-Galactose 4-Epimerase, Fucosidasen, G-Proteine, Galactosidasen, Glycosidasen, Glycosyltransferasen und Xylosidase, Antibiotika, Vitamin und Vitamin-Analoga, Hormone, DNA-Interkalatoren, Nucleoside, Nucleotide, Lektine, Vitamin B12, Lewis-X und Verwandte, Psoralene, Dientrienantibiotika, Carbacycline, VEGF (vascular endothelial growth factor), Somatostatin und dessen Derivate, Biotin-Derivate, Antihormone, tumorspezifische Proteine und Synthetika, Polymere, die sich in sauren oder basischen Bereichen des Körpers anreichern (pHgesteuerte Verteilung), Myoglobine, Apomyoglobine etc., Neurotransmitter-Peptide, Tumornecrosefaktoren, Peptide, die sich in entzündetem Gewebe anreichern, Bloodpool-Reagenzien, Anionen und Kationen-Transporterproteine, Polyester (z.B. der Milchsäure), Polyamide und Polyphosphate.

10. Verfahren zur Herstellung von Verbindungen der Formel VIIa und VIIb nach Anspruch 1, **dadurch gekennzeichnet, dass** man in an sich bekannter Weise in Verbindungen der allgemeinen Formel VII'a und VII'b 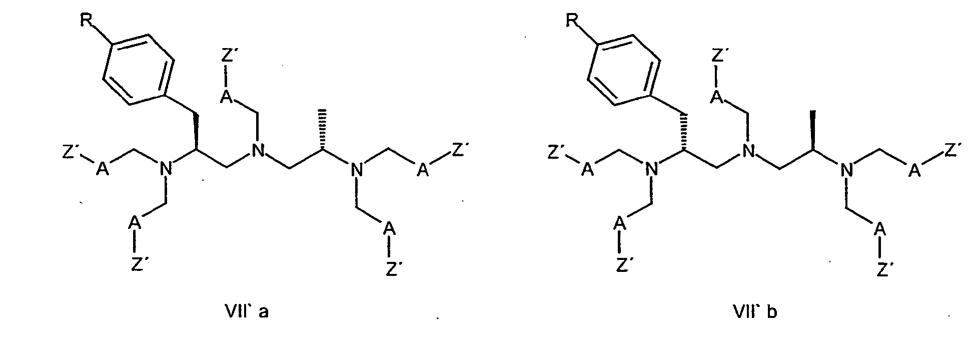 worin Z' eine Carboxylschutzgruppe bedeutet, die Schutzgruppen Z' abspaltet und die so erhaltenen Säuren in an sich bekannter Weise mit mindestens einem Metalloxid oder Metallsalz eines Elements der Ordnungszahlen 21-29, 31, 32, 37-39, 42-44, 46, 47, 49, 58-71, 75, 77, 82 oder 83 umsetzt und anschließend, falls gewünscht, vorhandene acide Wasserstoffatome mit anorganischen und/oder organischen oder Aminosäuren in physiologisch verträgliche Salze überführt.

11. Pharmazeutische Mittel enthaltend mindestens eine physiologisch verträgliche Verbindung nach Anspruch 2.

12. Pharmazeutische Mittel enthaltend mindestens eine physiologisch verträgliche Verbindung nach Anspruch 3.

13. Verwendung einer Verbindung nach Anspruch 2 zur Herstellung von Mitteln für die NMR-Diagnostik.

14. Verwendung einer Verbindung nach Anspruch 3 zur Herstellung von Mitteln für die Radiodiagnostik oder Radiotherapie.

15. Kit zur Herstellung von Radiopharmaka, umfassend eine Verbindung nach Anspruch 1, worin Z ein Wasserstoffatom ist, und eine Verbindung eines radioaktiven Elements der Ordnungszahlen 26, 27, 29, 31, 32, 37-39, 43, 46, 61, 62, 64, 67, 70, 71, 75, 77, 82 und 83.

## Claims

1. Compounds of the general formulae VIIa and VIIb 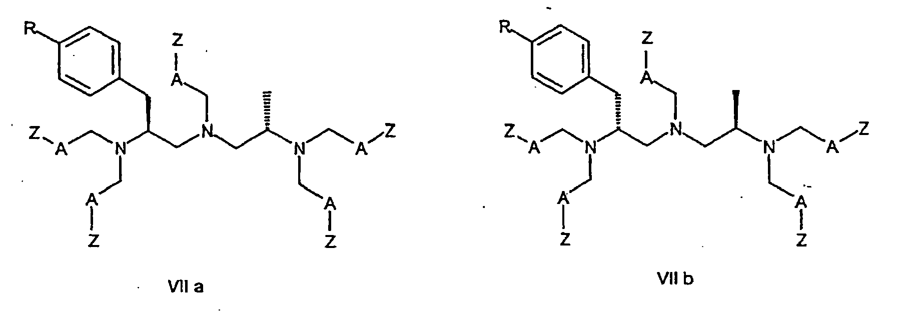 wherein
Z represents a hydrogen atom or a metal ion equivalent of an element of atomic number 21-29, 31, 32, 37-39, 42-44, 46, 47, 49, 58-71, 75, 77, 82 or 83,
A represents a -COO- group,
R represents a nitro group, an amino group or another functional group that can be linked to a biomolecule, or R represents a straight-chained or branched, saturated or unsaturated C₁-C₂₅-alkyl radical which is optionally interrupted by from one to six O atoms, or phenylene, -NHCO-, -CONH-, 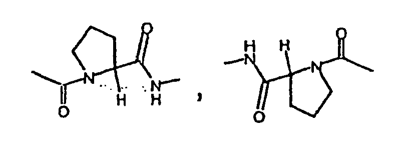 and/or -NH-(C=S)-NH- groups and which is optionally substituted in any desired position by from one to six carboxyl groups, hydroxyl groups, amino groups or other functional groups, as well as salts thereof with organic or inorganic bases, with the provisos that the alkyl radical contains at least one functional group that can be linked to a biomolecule, and that at least two radicals Z represent a metal ion equivalent.

2. Compounds according to claim 1, wherein at least two of the radicals Z represent a metal ion equivalent of a paramagnetic element of atomic numbers 21-29, 42, 44 and 58-70.

3. Compounds according to claim 1, wherein at least two of the radicals Z represent a metal ion equivalent of a radioactive element of atomic numbers 26, 27, 29, 31, 32, 37-39, 43, 46, 47, 49, 61, 62, 64, 70, 71, 75, 77, 82 and 83.

4. Compounds according to claim 1, wherein R represents a radical

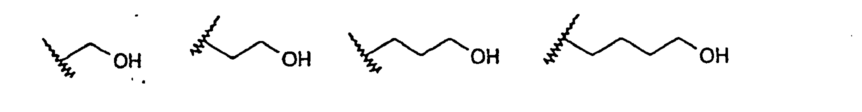

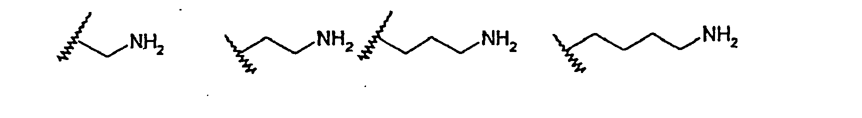

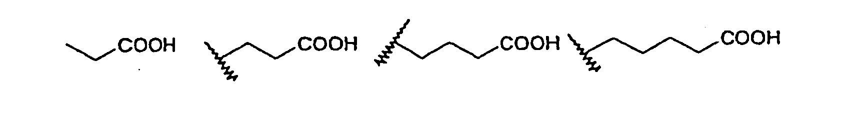

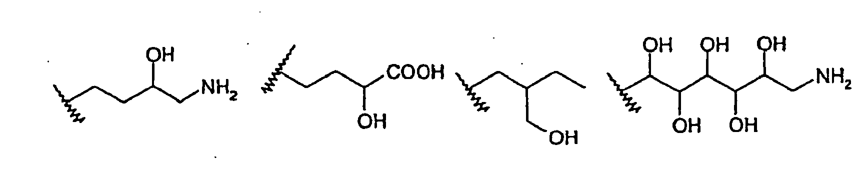

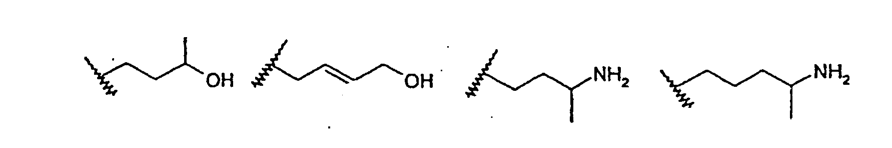

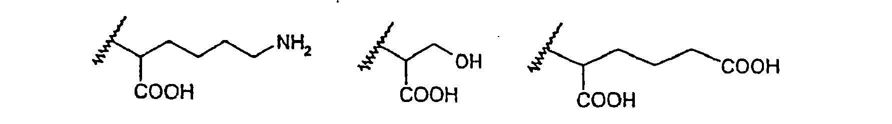

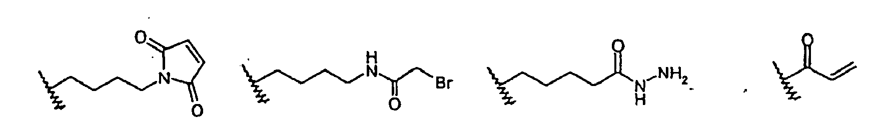

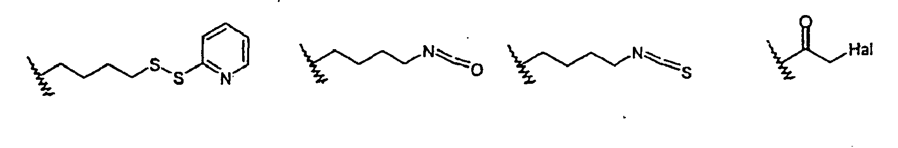

5. Compounds according to claim 1, wherein R represents a radical

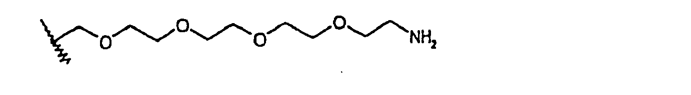

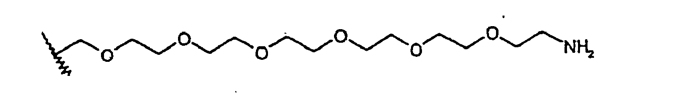

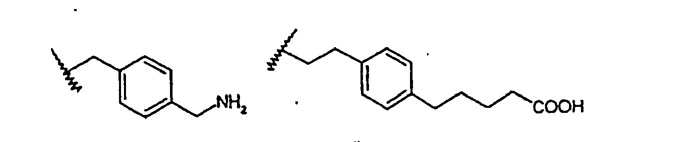

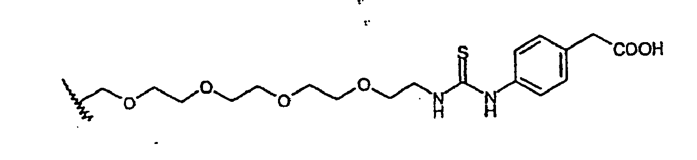

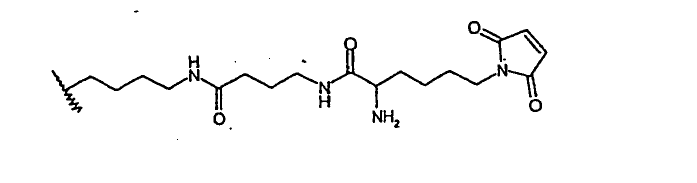

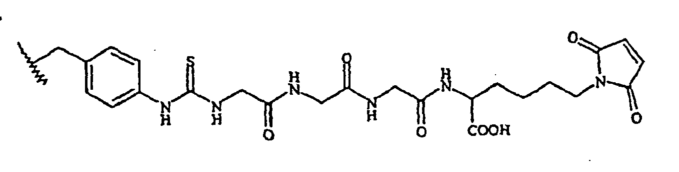

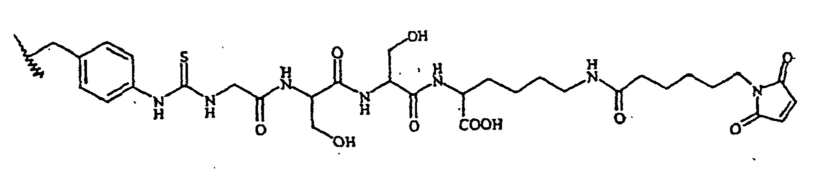

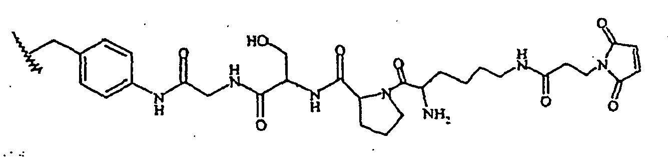

6. Compounds according to claim 1, wherein R represents a functional group carboxyl, activated carboxyl, amino, nitro, isocyanate, isothiocyanate, hydrazine, semicarbazide, thiosemicarbazide, chloroacetamide, bromoacetamide, iodoacetamide, acryl, acylamino, mixed anhydrides, azide, acid chloride, acid bromide, hydroxide, sulfonyl chloride, vinylsulfone, carbodiimide, maleimide or diazo.

7. Compounds according to claim 6, wherein the activated carboxyl group is selected from 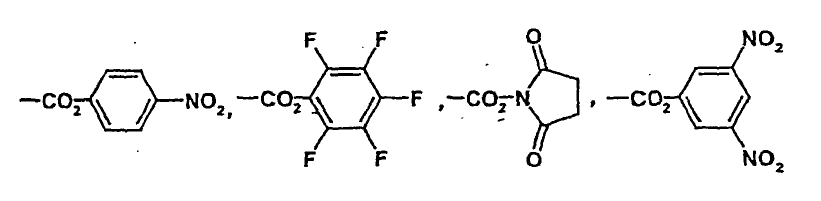 and

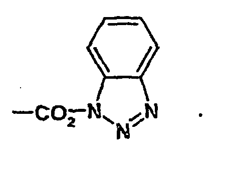

8. Use of compounds of the general formula VIIa or VIIb according to claim 1 in the preparation of conjugates with a biomolecule.

9. Use according to claim 8, wherein the biomolecule is selected from the group consisting of:
biopolymers, proteins, such as proteins having a biological function, HSA, BSA, etc., proteins and peptides that accumulate at specific sites in the organism (e.g. at receptors, cell membranes, channels, etc.), peptides cleavable by proteases, peptides having synthetic intended breaking points (e.g. labile esters, amides, etc.), peptides cleaved by metalloproteases, peptides having photocleavable linkers, peptides having groups cleavable by oxidative agents (oxidases), peptides having natural and non-natural amino acids, glycoproteins (glycopeptides), signal proteins, antiviral proteins and apoctosis, synthetically modified biopolymers, such as biopolymers derivatised with linkers, modified metalloproteases and derivatised oxidases, etc., carbohydrates (mono- to poly-saccharides), such as derivatised sugars, sugars cleavable in the organism, cyclodextrins and derivatives thereof, amino sugars, chitosan, polysulfates and acetylneuraminic acid derivatives, antibodies, such as monoclonal antibodies, antibody fragments, polyclonal antibodies, minibodies, single chains (including those linked to linkers to form multiple fragments), red blood corpuscles and other constituents of blood, cancer markers (e.g. CAA) and cell adhesion substances (e.g. Lewis X and anti-Lewis X derivatives), DNA and RNA fragments, such as derivatised DNAs and RNAs (e.g. those found by the SELEX process), synthetic RNA and DNA (also with non-natural bases), PNAs (Hoechst) and antisense, β-amino acids (Seebach), vector amines for insertion into the cell, biogenic amines, pharmaceuticals, oncological preparations, synthetic polymers directed at a biological target (e.g. receptor), steroids (natural and modified), prostaglandins, taxol and derivatives thereof, endothelins, alkaloids, folic acid and derivatives thereof, bioactive lipids, fats, fatty acid esters, synthetically modified mono-, di- and tri-glycerides, liposomes derivatised at the surface, micelles from natural fatty acids or from perfluoroalkyl compounds, porphyrins, texaphrins, extended porphyrins, cytochromes, inhibitors, neuramidases, neuropeptides, immunomodulators, such as FK 506, CAPE and gliotoxin, endoglycosidases, substrates activated by enzymes, such as calmodolin kinase, casein kinase II, glutathione S-transferase, heparinase, matrix metalloproteases, β-insulin receptor kinase, UDP galactose 4-epimerase, fucosidases, G proteins, galactosidases, glycosidases, glycosyl transferases and xylosidase, antibiotics, vitamin and vitamin analogues, hormones, DNA intercalators, nucleosides, nucleotides, lectins, vitamin B12, Lewis X and related compounds, psoralenes, diene triene antibiotics, carbacyclins, VEGF (vascular endothelial growth factor), somatostatin and derivatives thereof, biotin derivatives, antihormones, tumour-specific proteins and synthetics, polymers that accumulate in acidic or basic regions of the body (pH-controlled distribution), myoglobins, apomyoglobins, etc., neurotransmitter peptides, tumour necrosis factors, peptides that accumulate in inflamed tissue, bloodpool reagents, anion and cation transporter proteins, polyesters (e.g. of lactic acid), polyamides and polyphosphates.

10. Process for the preparation of compounds of formulae VIIa and VIIb according to claim 1, **characterised in that** in compounds of the general formulae VII'a and VII'b 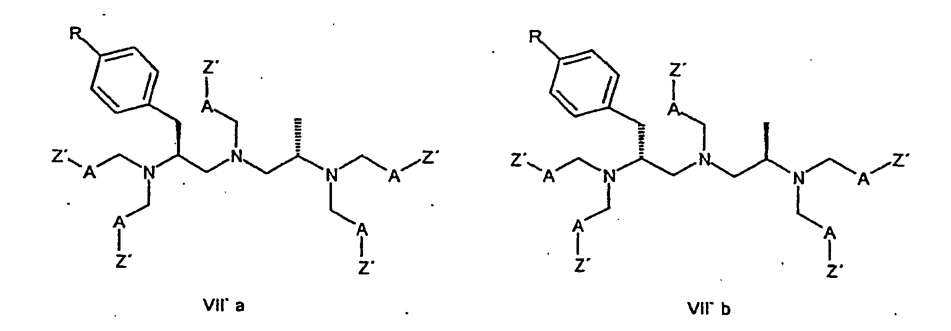 wherein Z' represents a carboxyl protecting group, the protecting groups Z' are cleaved in a manner known per se and the acids so obtained are reacted in a manner known *per se* with at least one metal oxide or metal salt of an element of atomic numbers 21-29, 31, 32, 37-39, 42-44, 46, 47, 49, 58-71, 75, 77, 82 or 83 and then, if desired, any acidic hydrogen atoms present are converted into physiologically acceptable salts using inorganic and/or organic amino acids.

11. Pharmaceutical agents comprising at least one physiologically acceptable compound according to claim 2.

12. Pharmaceutical agents comprising at least one physiologically acceptable compound according to claim 3.

13. Use of a compound according to claim 2 in the preparation of agents for NMR diagnostics.

14. Use of a compound according to claim 3 in the preparation of agents for radiodiagnostics or radiotherapy.

15. Kit for the preparation of radiopharmaceuticals, comprising a compound according to claim 1 wherein Z is a hydrogen atom and a compound of a radioactive element of atomic numbers 26, 27, 29, 31, 32, 37-39, 43, 46, 61, 62, 64, 67, 70, 71, 75, 77, 82 and 83.

## Revendications

1. Composés de formules générales VIIa et VIIb : 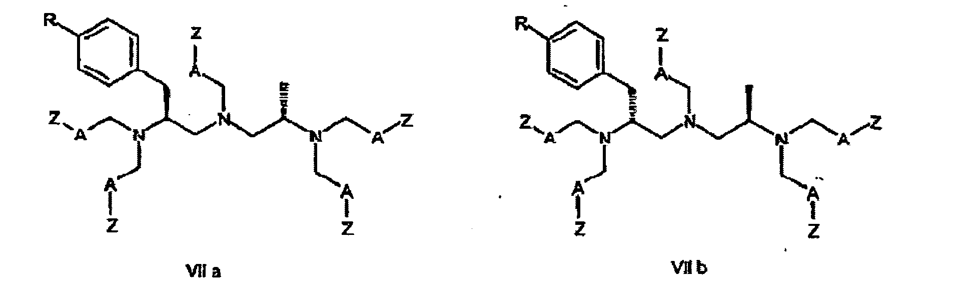 dans lesquelles :
Z est un atome d'hydrogène ou un équivalent ion métallique d'un élément de numéro atomique 21-29, 31, 32, 37-39, 42-44, 46, 47, 49, 58-71, 75, 77, 82 ou 83,
A est un groupement -COO-,
R est un groupement nitro, un groupement amine, ou un autre groupement fonctionnel qui peut être lié à une biomolécule ou un radical alkyle C₁-C₂₅ linéaire ou ramifié, saturé ou insaturé, et le cas échéant interrompu par un à six atomes d'oxygène, ou respectivement par un groupement phénylène, -NHCO-, - CONH-, 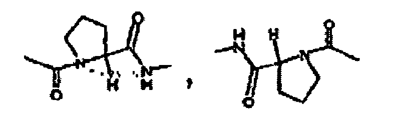 et/ou -NH-(C=S)-NH-, qui est substitué le cas échéant en une position au choix avec un jusqu'à six groupements carboxyles, hydroxyles, amines ou autres groupements fonctionnels, ainsi que leurs sels avec des bases organiques ou non organiques, avec les recommandations que le radical alkyle contienne au moins un groupement fonctionnel qui peut être lié à une biomolécule, et qu'au moins deux Z représentent un équivalent ion métallique.

2. Composés selon la revendication 1 dans lesquels au moins deux des radicaux Z sont des équivalents ions métalliques d'un élément paramagnétique de numéro atomique 21-29, 42, 44 et 58-70.

3. Composés selon la revendication 1 dans lesquels au moins deux des radicaux Z sont des équivalents ions métalliques d'un élément radioactif de numéro atomique 26, 27, 29, 31, 32, 37-39, 43, 46, 47, 49, 61, 62, 64, 70, 71, 75, 77, 82 et 83.

4. Composés selon la revendication 1 dans lesquels R est un radical :

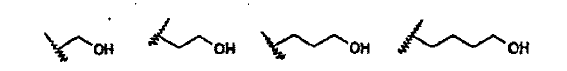

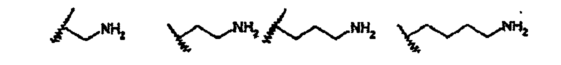

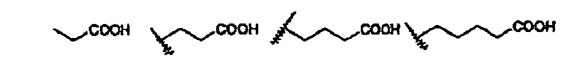

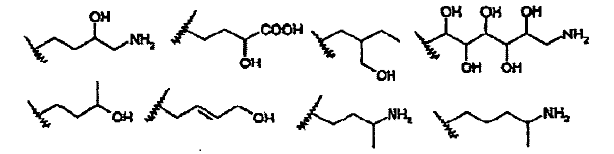

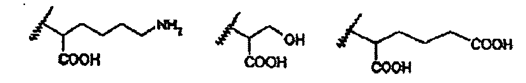

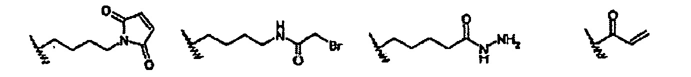

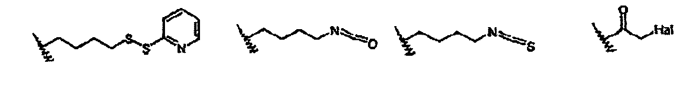

5. Composés selon la revendication 1 dans lesquels R est un radical :

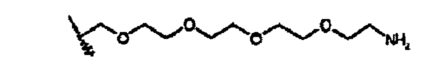

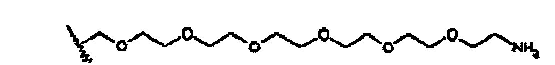

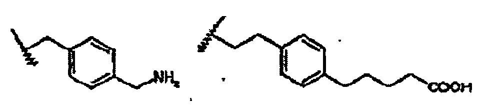

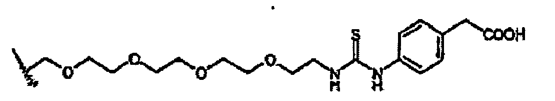

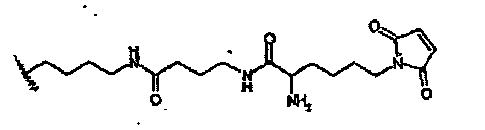

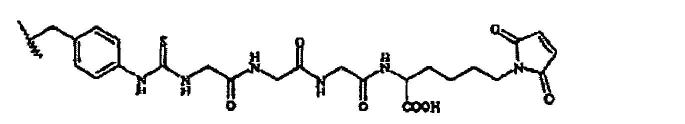

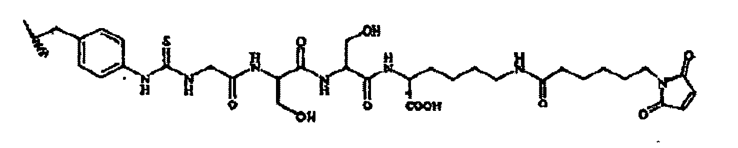

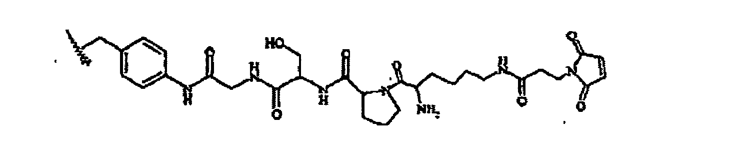

6. Composés selon la revendication 1 dans lesquels R est un groupement fonctionnel carboxyle, carboxyle activé, amine, nitro, isocyanate, isothiocyanate, hydrazine, semicarbazide, thiosemicarbazide, chloracétamide, bromacétamide, iodacétamide, acryle, acylamino, mélanges d'anhydrides, azides, chlorures d'acide, bromures d'acide, hydroxydes, chlorures de sulfonyle, vinylsulfones, carbodiimides, maléimides ou diazo.

7. Composés selon la revendication 6 dans lesquels le groupement carboxyle activé est choisi parmi : 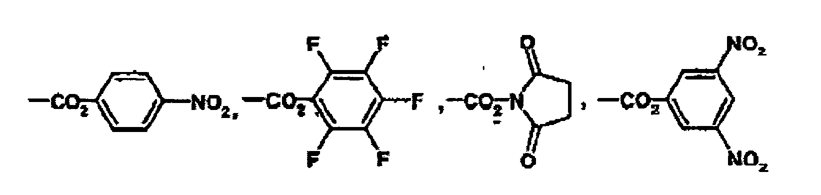 et

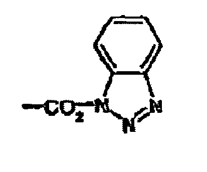

8. Utilisation de composés de formules générales VIIa et VIIb selon la revendication 1 pour la fabrication de conjugués avec une biomolécule.

9. Utilisation selon la revendication 8 dans laquelle la biomolécule est choisie dans le groupe composé de biopolymères, de protéines comme des protéines qui ont une fonction biologique, HSA, BSA, etc. des protéines et des peptides qui s'accumulent à des endroits déterminés dans l'organisme (ex. sur des récepteurs, des membranes cellulaires, des canaux, etc.), des peptides que l'on peut couper par des protéases, des peptides avec sites synthétiques destinés à la rupture (ex. esters labiles, amides, etc.), des peptides coupées par des métalloprotéases, des peptides avec des liaisons photodivables, des peptides avec des groupements que l'on peut couper par des moyens d'oxydation (oxydases), des peptides avec des acides aminés naturels et artificiels, des glycoprotéines (glycopeptides), des protéines signal, des protéines antivirales et d'apoptose, des biopolymères synthétiquement modifiés comme des biopolymères modifiés avec des liants, des métalloprotéases modifiés et des oxydases modifiés, etc. des hydrates de carbone (mono- et polysaccharides) comme des sucres modifiés, des sucres dégradables dans l'organisme, des cyclodextrines et leurs dérivés, des aminosucres, des chitosanes, des polysulfates et des dérivés d'acétylneuraminoacides, des anticorps comme des anticorps monoclonaux, des fragments d'anticorps, des anticorps polyclonaux, des mini corps *(minibodies),* des chaînes simples (y compris celles qui sont liées par des liants à des fragments multiples), des globules rouges et autres composants sanguins, des marqueurs du cancer (ex. CAA), et des substances d'adhésion cellulaire (ex. Lewis X et dérivés anti-Lewis X), des fragments d'ADN et d'ARN, comme des ADN et ARN modifiés, ceux qui ont été trouvés par procédé SELEX), des ADN et ARN synthétiques (y compris à base naturelle), des aromatiques polynucléaires (Hoechst) et des antisens, des acides β aminés (Seebach), des amines vectrices pour l'incorporation dans la cellule, des amines biogéniques, des produits pharmaceutiques, des préparations oncologiques, des polymères synthétiques qui sont dirigés contre une cible biologique (ex. récepteur), des stéroïdes (naturels et modifiés), la prostaglandine, le taxol et ses dérivés, l'endothéline, des alcaloïdes, l'acide folique et ses dérivés, des lipides bioactifs, des graisses, des esters d'acides gras, des mono-, bi- ou triglycérides synthétiquement modifiés, des liposomes modifiés en surface, des micelles d'acides gras naturels ou de composés perfluoroalkyles, la porphyrine, la texaphrine, des porphyrines élargies, des cytochromes, des inhibiteurs, des neuramidases, des neuropeptides, des immunomodulateurs comme FK 506, CAPE et des gliotoxines, des endoglycosidases, des substrats activés par des enzymes comme la calmodoline kinase, la caséine kinase II, la glutathion S transférase, l'héparinase, des métalloprotéases de matrice, la β-insuline récepteur kinase, l'UDP-galactose-4 épimérase, des fucosidases, des G-protéines, des galactosidases, des glycosidases, des glycosyltransférases et des xylosidases, des antibiotiques, des vitamines et des analogues de vitamines, des hormones, des intercalateurs ADN, des nucléosides, des nucléotides, des lectines, la vitamine B12, Lewis X et molécules parentes, le psoralène, des antibiotiques diène-triène, la carbacycline, le facteur de croissance endothelial vasculaire (VEGF), la somatostatine et ses dérivés, des dérivés de la biotine, des anti-hormones, des protéines spécifiques de tumeurs et des produits de synthèse, des polymères qui s'accumulent dans des régions acides ou basiques du corps (répartition contrôlée par le pH), la myoglobine, l'apomyoglobine, etc., des peptides neurotransmetteurs, des facteurs de nécrose tumorale, des peptides qui s'accumulent dans les tissus inflammatoires, des réactifs bloodpool, des protéines de transport d'anions ou de cations, des polyesters (par ex. de l'acide lactique), des polyamides et des polyphosphates.

10. Procédé de fabrication de composés de formules générales VIIa et VIIb selon la revendication 1, **caractérisé en ce que** l'on découpe les groupements protecteurs Z' d'une manière connue dans des composés de formules générales VII'a et VII'b : 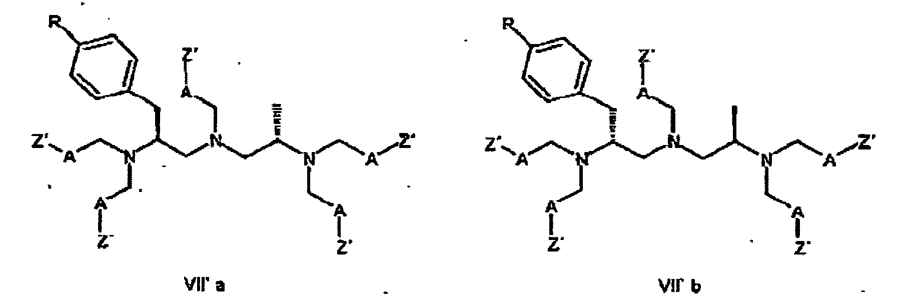 où Z' représente un groupement protecteur carboxyle, et ou transforme d'une manière connue en soi les acides ainsi produits avec au moins un oxyde métallique ou sel métallique d'un élément de numéro atomique 21-29, 31, 32, 37-39, 42-44, 46, 47, 49, 58-71, 75, 77, 82 ou 83, et ensuite, si on le veut, on transforme des atomes d'hydrogène acides présents avec des acides aminés non organiques et/ou organiques en sels physiologiquement compatibles.

11. Agent pharmaceutique contenant au moins un composé physiologiquement compatible selon la revendication 2.

12. Agent pharmaceutique contenant au moins un composé physiologiquement compatible selon la revendication 3.

13. Utilisation d'un composé selon la revendication 2 pour la fabrication d'agents pour le diagnostic RMN.

14. Utilisation d'un composé selon la revendication 3 pour la fabrication d'agents pour le radiodiagnostic ou la radiothérapie.

15. Ensemble de fabrication de produits radiopharmaceutiques comprenant un composé selon la revendication 1, dans lequel Z est un atome d'hydrogène, et un composé d'un élément radioactif de numéro atomique 26, 27, 29, 31, 32, 37-39, 43, 46, 61, 62, 64, 67, 70, 71, 75, 77, 82 et 83.
